# EUROPEAN PATENT APPLICATION

(11) **EP 4 302 778 A1**
(43) Date of publication of application: **10.01.2024**
(21) Application number: 22762538.1
(22) Date of filing: 02.03.2022
(51) Int. Cl.: A61K 39/395, C07K 16/24, C12N 15/13, A61P 37/02

(54) **PHARMACEUTICAL COMPOSITION CONTAINING ANTI-TSLP ANTIBODY**

(30) Priority: 03.03.2021 CN 202110235660
(71) Applicant: CHIA TAI TIANQING PHARMACEUTICAL GROUP CO., LTD., Lianyungang, Jiangsu 222062 (CN)
(72) Inventor: KONG, Lingjie, Nanjing, Jiangsu 211100 (CN); CHENG, Yanju, Nanjing, Jiangsu 211100 (CN); GUO, Xiaolu, Nanjing, Jiangsu 211100 (CN)
(74) Representative: Grünecker Patent- und Rechtsanwälte PartG mbB
(86) International application number: PCT/CN2022/078718
(87) International publication number: WO 2022/184074

(57) **Abstract**

Provided are a pharmaceutical composition containing an anti-TSLP antibody, and a use thereof. In particular, provided are a pharmaceutical composition, containing (a) an anti-TSLP antibody, (b) a buffer, (c) a surfactant, and one or more (d) stabilizers, and a use of the pharmaceutical composition in preparation of a medicament for treating and preventing TSLP-related diseases.

## Description

All documents cited or referenced in the present application (including but not limited to documents, patents, patent applications) and all documents cited or referenced in the documents cited in the present application, as well as any manufacturer's instructions, descriptions, product specifications, and product sheets for any products mentioned in the present application or any document incorporated by reference, are incorporated by reference and can be used in the practice of the present disclosure. More specifically, all references cited herein are incorporated by reference to the same extent as if each reference is specifically and individually indicated to be incorporated by reference in the present application. Moreover, in any country or region, any reference to these publications herein is not to be construed as an admission that the publications form part of the commonly recognized knowledge in the art.

### TECHNICAL FIELD

The present disclosure belongs to the field of pharmaceutical formulations, and in particular, the present disclosure relates to a stable pharmaceutical composition comprising an anti-TSLP antibody. Alternatively, the present disclosure relates to a stable pharmaceutical composition comprising an anti-TSLP antibody at a high concentration.

### BACKGROUND

Thymic stromal lymphopoietin (TSLP) is a cytokine produced from epithelial cells. It is closely related to IL-7 and binds to TSLPR (a heterodimer of the IL-7 receptor α chain and the TSLP receptor chain). TSLP induces DC cell polarization during the induction phase of the immune response, promotes differentiation of helper T cells (Th)2 and production of Th2 cytokines, and in addition, TSLP can also directly promote T cell proliferation and increase secretion of Th2 cytokines. Therefore, TSLP is considered to be a major regulator of Th2-driven inflammation, and upregulation of TSLP is related to the pathogenesis of Th2 cell-related diseases such as atopic dermatitis and asthma (Rui He *et al*., (2010) supra; Ito T et al., (2005) The Journal of Experimental Medicine 202(9): 1213-1223; He R et al., (2008) Proc Natl Acad Sci USA 105(33):11875-11880). In another aspect, TSLP mediates several immune homeostasis in the gut and thymus. For example, under bacterial stimulation, TSLP is upregulated in a strain-dependent manner in the intestinal epithelial cell line, acting synergistically with transforming growth factor β to promote Treg cell differentiation. TSLP can also be produced from human primary intestinal epithelial cells for modulating CD103⁺ DC cells to become a tolerogenic phenotype (Katerina Tsilingiri et al., (2017) Cellular and Molecular Gastroenterology and Hepatology 3(2): 174-182; Zeuthen LH et al., (2008) Immunology 123:197-208; Iliev ID et al., (2009) Gut 58:1481-1489). The dual role of TSLP on the immune system leads to the discovery of two isoforms, a long isoform and a short isoform, where the short isoform composed of the last 63 amino acid residues of the longer one. These two isoforms are controlled by different promoters and are expressed depending on environment, tissue and stimulus (Harada M et al., (2011) American Journal of Respiratory Cell and Molecular Biology 44:787-793). Long isoform expression was upregulated while the short isoform expression was downregulated in human intestinal epithelial cells in response to highly immunogenic microbial strains, whereas the opposite expression pattern was observed after challenge with a commensal E. coli strain. The expression pattern of TSLP isoforms has also been studied in several TSLP-related diseases. For example, over-expression of long TSLP is observed in asthma, ulcerative colitis, atopic dermatitis and psoriasis, while reduced expression of long TSLP is found in gluten-induced enteropathy. Expression of short TSLP is down-regulated in Crohn's disease, gluten-induced enteropathy and atopic dermatitis (Katerina Tsilingiri *et al*., (2017) supra; Fornasa G et al., (2015) J Allergy Clin Immunol 136:413-422). TSLP emerges as a clinical target because of its relevance to a variety of diseases. There will be an increasing number of anti-TSLP antibodies developed and applied. Therefore, there is a need in the art to develop a pharmaceutical composition comprising an anti-TSLP antibody so that the anti-TSLP antibody is suitable for production and administration to patients and retains biological activity and stability during storage and subsequent use.

### SUMMARY

The present disclosure provides a pharmaceutical composition comprising an anti-TSLP antibody, the pharmaceutical composition being capable of achieving a stable effect and thus being suitable for administration in a subject.

In particular, the present disclosure provides a pharmaceutical composition comprising an anti-TSLP antibody, such as: (i) a pharmaceutical composition capable of inhibiting the formation of high-molecular impurities; (ii) a pharmaceutical composition capable of inhibiting the production of charge variants; (iii) a pharmaceutical composition capable of maintaining the biological activity of an antibody; and/or (iv) a pharmaceutical composition capable of reducing the increased viscosity when the antibody has a relatively high concentration.

The present disclosure provides a pharmaceutical composition comprising: (a) an anti-TSLP antibody, (b) a buffering agent, (c) a surfactant, and one or more (d) stabilizers.

In some embodiments, the anti-TSLP antibody in the aforementioned pharmaceutical composition has a concentration of 30 mg/mL to 300 mg/mL, preferably 50 mg/mL to 250 mg/mL, preferably 70 mg/mL to 200 mg/mL, more preferably 90 mg/mL to 150 mg/mL. In some embodiments, the anti-TSLP antibody in the aforementioned pharmaceutical composition has a concentration of about 120 mg/mL. In some embodiments, the anti-TSLP antibody in the aforementioned pharmaceutical composition has a concentration of about 130 mg/mL. In some embodiments, the anti-TSLP antibody in the aforementioned pharmaceutical composition has a concentration of about 140 mg/mL. In some embodiments, the anti-TSLP antibody in the aforementioned pharmaceutical composition has a concentration of about 150 mg/mL. In some embodiments, the anti-TSLP antibody in the aforementioned pharmaceutical composition has a concentration including, but not limited to: about 90 mg/mL, about 100 mg/mL, about 110 mg/mL, about 115 mg/mL, about 120 mg/mL, about 125 mg/mL, about 130 mg/mL, about 135 mg/mL, about 140 mg/mL, about 145 mg/mL, or about 150 mg/mL.

In some embodiments, the buffering agent in the aforementioned pharmaceutical composition comprises a phosphate buffering agent, a histidine buffering agent, an acetate buffering agent, or a citrate buffering agent, etc. In some embodiments, the buffering agent in the aforementioned pharmaceutical composition comprises a phosphate buffering agent. In some embodiments, the buffering agent in the aforementioned pharmaceutical composition comprises an acetate buffering agent. Preferably, the buffering agent in the aforementioned pharmaceutical composition comprises a histidine buffering agent.

In some specific embodiments, the phosphate buffering agent comprises a sodium phosphate buffering agent or a potassium phosphate buffering agent, the acetate buffering agent comprises a sodium acetate buffering agent, a potassium acetate buffering agent or an ammonium acetate buffering agent, and the citrate buffering agent comprises a sodium citrate buffering agent, a potassium citrate buffering agent or a calcium citrate buffering agent. In some embodiments, the buffering agent in the aforementioned pharmaceutical composition comprises a sodium phosphate buffering agent, for example, a sodium phosphate buffering agent consisting of disodium hydrogen phosphate and sodium dihydrogen phosphate. In some embodiments, the buffering agent in the aforementioned pharmaceutical composition comprises a sodium acetate buffering agent, for example, a sodium acetate buffering agent consisting of sodium acetate and acetic acid. In some embodiments, the buffering agent in the aforementioned pharmaceutical composition comprises a sodium citrate buffering agent, for example, a sodium citrate buffering agent consisting of citric acid and sodium citrate.

In some embodiments, the buffering agent in the aforementioned pharmaceutical composition has a concentration of 1 mM to 100 mM, preferably 2 mM to 80 mM, preferably 5 mM to 60 mM, more preferably 10 mM to 40 mM. In some embodiments, the buffering agent in the aforementioned pharmaceutical composition has a concentration of about 10 mM. In some embodiments, the buffering agent in the aforementioned pharmaceutical composition has a concentration of about 18 mM. In some embodiments, the buffering agent in the aforementioned pharmaceutical composition has a concentration of about 20 mM. In some embodiments, the buffering agent in the aforementioned pharmaceutical composition has a concentration of about 22 mM. In some embodiments, the buffering agent in the aforementioned pharmaceutical composition has a concentration including, but not limited to: about 10 mM, about 11 mM, about 12 mM, about 13 mM, about 14 mM, about 15 mM, about 16 mM, about 17 mM, about 18 mM, about 19 mM, about 20 mM, about 21 mM, about 22 mM, about 23 mM, about 24 mM, about 25 mM, about 26 mM, about 27 mM, about 28 mM, about 30 mM, about 32 mM, about 34 mM, about 36 mM, about 38 mM, or about 40 mM.

In some embodiments, the buffering agent in the aforementioned pharmaceutical composition has a pH of 5 to 7, preferably 5.5 to 7, more preferably 5.5 to 6.5. In some embodiments, the buffering agent in the aforementioned pharmaceutical composition has a pH of about 5.6. In some embodiments, the buffering agent in the aforementioned pharmaceutical composition has a pH of about 5.8. In some embodiments, the buffering agent in the aforementioned pharmaceutical composition has a pH of about 6. In some embodiments, the buffering agent in the aforementioned pharmaceutical composition has a pH including, but not limited to: about 5, about 5.1, about 5.2, about 5.3, about 5.4, about 5.5, about 5.6, about 5.7, about 5.8, about 5.9, about 6, about 6.1, about 6.2, about 6.3, about 6.4, or about 6.5.

In some embodiments, the surfactant in the aforementioned pharmaceutical composition is selected from polysorbates (e.g., polysorbate 20, polysorbate 40, polysorbate 60, polysorbate 65, polysorbate 80, polysorbate 81, polysorbate 85), poloxamers (e.g., poloxamer 181, poloxamer 188, poloxamer 407), polyethylene glycols, poloxamers, and the like. In some embodiments, the surfactant in the aforementioned pharmaceutical composition is polysorbate 80. In some embodiments, the surfactant in the aforementioned pharmaceutical composition is polysorbate 20. In some embodiments, the surfactant in the aforementioned pharmaceutical composition has a concentration of 0.001% (w/v) to 0.1% (w/v), preferably 0.004% (w/v) to 0.08% (w/v), preferably 0.006% (w/v) to 0.06% (w/v), more preferably 0.008% (w/v) to 0.04% (w/v). In some embodiments, the surfactant in the aforementioned pharmaceutical composition has a concentration of about 0.01% (w/v). In some embodiments, the surfactant in the aforementioned pharmaceutical composition has a concentration of about 0.02% (w/v). In some embodiments, the surfactant in the aforementioned pharmaceutical composition has a concentration of about 0.04% (w/v). In some embodiments, the surfactant in the aforementioned pharmaceutical composition has a concentration including, but not limited to: about 0.008% (w/v), about 0.009% (w/v), about 0.01% (w/v), about 0.02% (w/v), about 0.03% (w/v), about 0.04% (w/v), about 0.05% (w/v), or about 0.06% (w/v).

In some embodiments, the stabilizer in the aforementioned pharmaceutical composition comprises trehalose, mannitol, sucrose, arginine or a pharmaceutically acceptable salt thereof, glycine, or proline. In some embodiments, the stabilizer in the aforementioned pharmaceutical composition comprises mannitol. In some embodiments, the stabilizer in the aforementioned pharmaceutical composition comprises arginine. In some embodiments, the stabilizer in the aforementioned pharmaceutical composition comprises proline. In some embodiments, the aforementioned pharmaceutical composition comprises two stabilizers, such as sucrose and proline. The arginine or the pharmaceutically acceptable salt thereof comprises arginine, arginine hydrochloride or arginine acetate, etc.

In some embodiments, the stabilizer in the aforementioned pharmaceutical composition has a concentration of 100 mM to 1000 mM, 120 mM to 800 mM, preferably 150 mM to 700 mM, more preferably 150 mM to 600 mM. In some embodiments, the stabilizer in the aforementioned pharmaceutical composition has a concentration of about 200 mM. In some embodiments, the stabilizer in the aforementioned pharmaceutical composition has a concentration of about 250 mM. In some embodiments, the stabilizer in the aforementioned pharmaceutical composition has a concentration of about 400 mM. In some embodiments, the stabilizer in the aforementioned pharmaceutical composition has a concentration of about 500 mM. In some embodiments, the stabilizer in the aforementioned pharmaceutical composition has a concentration including, but not limited to: about 120 mM, about 150 mM, about 200 mM, about 210 mM, about 230 mM, about 250 mM, about 270 mM, about 290 mM, about 300 mM, about 310 mM, about 330 mM, about 350 mM, about 370 mM, about 380 mM, about 390 mM, about 400 mM, about 410 mM, about 420 mM, about 430 mM, about 450 mM, about 470 mM, about 490 mM, about 500 mM, about 510 mM, about 530 mM, about 550 mM, about 570 mM, or about 600 mM.

In some embodiments, the aforementioned pharmaceutical composition has a pH of 5 to 7, preferably 5.5 to 7, more preferably 5.5 to 6.5. In some embodiments, the aforementioned pharmaceutical composition has a pH of about 5.6. In some embodiments, the aforementioned pharmaceutical composition has a pH of about 5.8. In some embodiments, the aforementioned pharmaceutical composition has a pH of about 6. In some embodiments, the aforementioned pharmaceutical composition has a pH including, but not limited to: about 5, about 5.1, about 5.2, about 5.3, about 5.4, about 5.5, about 5.6, about 5.7, about 5.8, about 5.9, about 6, about 6.1, about 6.2, about 6.3, about 6.4, or about 6.5.

In some embodiments, the aforementioned pharmaceutical composition comprises: (a) about 30 mg/mL to about 300 mg/mL of an anti-TSLP antibody, (b) about 1 mM to about 100 mM of a buffering agent, (c) about 0.001% (w/v) to about 0.1% (w/v) of a surfactant, and one or more (d) about 100 mM to about 1000 mM of stabilizers; wherein the pharmaceutical composition has a pH of about 5 to about 7, preferably about 5.5 to about 7, more preferably about 5.5 to about 6.5.

In some embodiments, the aforementioned pharmaceutical composition comprises: (a) about 50 mg/mL to about 250 mg/mL of an anti-TSLP antibody, (b) about 2 mM to about 80 mM of a buffering agent, (c) about 0.004% (w/v) to about 0.08% (w/v) of a surfactant, and one or more (d) about 120 mM to about 800 mM of stabilizers; wherein the pharmaceutical composition has a pH of about 5 to about 7, preferably about 5.5 to about 7, more preferably about 5.5 to about 6.5.

In some embodiments, the aforementioned pharmaceutical composition comprises: (a) about 70 mg/mL to about 200 mg/mL of an anti-TSLP antibody, (b) about 5 mM to about 60 mM of a buffering agent, (c) about 0.006% (w/v) to about 0.06% (w/v) of a surfactant, and one or more (d) about 150 mM to about 700 mM of stabilizers; wherein the pharmaceutical composition has a pH of about 5 to about 7, preferably about 5.5 to about 7, more preferably about 5.5 to about 6.5.

In some embodiments, the aforementioned pharmaceutical composition comprises: (a) about 90 mg/mL to about 150 mg/mL of an anti-TSLP antibody, (b) about 10 mM to about 40 mM of a buffering agent, (c) about 0.008% (w/v) to about 0.04% (w/v) of a surfactant, and one or more (d) about 150 mM to about 600 mM of stabilizers; wherein the pharmaceutical composition has a pH of about 5 to about 7, preferably about 5.5 to about 7, more preferably about 5.5 to about 6.5.

In some embodiments, the aforementioned pharmaceutical composition comprises: (a) about 120 mg/mL to about 150 mg/mL of an anti-TSLP antibody, (b) about 10 mM to about 40 mM of a buffering agent, (c) about 0.008% (w/v) to about 0.04% (w/v) of a surfactant, and one or more (d) about 150 mM to about 600 mM of stabilizers; wherein the pharmaceutical composition has a pH of about 5 to about 7, preferably about 5.5 to about 7, more preferably about 5.5 to about 6.5.

In some embodiments, the aforementioned pharmaceutical composition comprises: (a) about 30 mg/mL to about 300 mg/mL of an anti-TSLP antibody, (b) about 1 mM to about 100 mM of a phosphate buffering agent, a histidine buffering agent or an acetate buffering agent, (c) about 0.001% (w/v) to about 0.1% (w/v) of polysorbate 20 or polysorbate 80, and (d) about 100 mM to about 1000 mM of trehalose, mannitol or sucrose, and/or about 100 mM to about 1000 mM of arginine or a pharmaceutically acceptable salt thereof, glycine, or proline; wherein the pharmaceutical composition has a pH of about 5 to about 7, preferably about 5.5 to about 7, more preferably about 5.5 to about 6.5.

In some embodiments, the aforementioned pharmaceutical composition comprises: (a) about 50 mg/mL to about 250 mg/mL of an anti-TSLP antibody, (b) about 2 mM to about 80 mM of a phosphate buffering agent, a histidine buffering agent or an acetate buffering agent, (c) about 0.004% (w/v) to about 0.08% (w/v) of polysorbate 20 or polysorbate 80, and (d) about 120 mM to about 800 mM of trehalose, mannitol or sucrose, and/or about 120 mM to about 800 mM of arginine or a pharmaceutically acceptable salt thereof, glycine, or proline; wherein the pharmaceutical composition has a pH of about 5 to about 7, preferably about 5.5 to about 7, more preferably about 5.5 to about 6.5.

In some embodiments, the aforementioned pharmaceutical composition comprises: (a) about 70 mg/mL to about 200 mg/mL of an anti-TSLP antibody, (b) about 5 mM to about 60 mM of a phosphate buffering agent, a histidine buffering agent or an acetate buffering agent, (c) about 0.006% (w/v) to about 0.06% (w/v) of polysorbate 20 or polysorbate 80, and (d) about 150 mM to about 700 mM of trehalose, mannitol or sucrose, and/or about 150 mM to about 700 mM of arginine or a pharmaceutically acceptable salt thereof, glycine, or proline; wherein the pharmaceutical composition has a pH of about 5 to about 7, preferably about 5.5 to about 7, more preferably about 5.5 to about 6.5.

In some embodiments, the aforementioned pharmaceutical composition comprises: (a) about 90 mg/mL to about 150 mg/mL of an anti-TSLP antibody, (b) about 10 mM to about 40 mM of a phosphate buffering agent, a histidine buffering agent or an acetate buffering agent, (c) about 0.008% (w/v) to about 0.04% (w/v) of polysorbate 20 or polysorbate 80, and (d) about 150 mM to about 600 mM of trehalose, mannitol or sucrose, and/or about 150 mM to about 600 mM of arginine or a pharmaceutically acceptable salt thereof, glycine, or proline; wherein the pharmaceutical composition has a pH of about 5 to about 7, preferably about 5.5 to about 7, more preferably about 5.5 to about 6.5.

In some embodiments, the aforementioned pharmaceutical composition comprises: (a) about 120 mg/mL to about 150 mg/mL of an anti-TSLP antibody, (b) about 10 mM to about 40 mM of a phosphate buffering agent, a histidine buffering agent or an acetate buffering agent, (c) about 0.008% (w/v) to about 0.04% (w/v) of polysorbate 20 or polysorbate 80, and (d) about 150 mM to about 600 mM of trehalose, mannitol or sucrose, and/or about 150 mM to about 600 mM of arginine or a pharmaceutically acceptable salt thereof, glycine, or proline; wherein the pharmaceutical composition has a pH of about 5 to about 7, preferably about 5.5 to about 7, more preferably about 5.5 to about 6.5.

In some embodiments, the aforementioned pharmaceutical composition comprises: (a) about 30 mg/mL to about 300 mg/mL of an anti-TSLP antibody, (b) about 1 mM to about 100 mM of a sodium phosphate buffering agent, a histidine buffering agent or a sodium acetate buffering agent, (c) about 0.001% (w/v) to about 0.1% (w/v) of polysorbate 80, and (d) about 100 mM to about 1000 mM of mannitol, and/or about 100 mM to about 1000 mM of arginine or a pharmaceutically acceptable salt thereof, or proline; wherein the pharmaceutical composition has a pH of about 5 to about 7, preferably about 5.5 to about 7, more preferably about 5.5 to about 6.5.

In some embodiments, the aforementioned pharmaceutical composition comprises: (a) about 50 mg/mL to about 250 mg/mL of an anti-TSLP antibody, (b) about 2 mM to about 80 mM of a sodium phosphate buffering agent, a histidine buffering agent or a sodium acetate buffering agent, (c) about 0.004% (w/v) to about 0.08% (w/v) of polysorbate 80, and (d) about 120 mM to about 800 mM of mannitol; and/or about 120 mM to about 800 mM of arginine or a pharmaceutically acceptable salt thereof, or proline; wherein the pharmaceutical composition has a pH of about 5 to about 7, preferably about 5.5 to about 7, more preferably about 5.5 to about 6.5.

In some embodiments, the aforementioned pharmaceutical composition comprises: (a) about 70 mg/mL to about 200 mg/mL of an anti-TSLP antibody, (b) about 5 mM to about 60 mM of a sodium phosphate buffering agent, a histidine buffering agent, or a sodium acetate buffering agent, (c) about 0.006% (w/v) to about 0.06% (w/v) of polysorbate 80, and (d) about 150 mM to about 700 mM of mannitol, and/or about 150 mM to about 700 mM of arginine or a pharmaceutically acceptable salt thereof, or proline; wherein the pharmaceutical composition has a pH of about 5 to about 7, preferably about 5.5 to about 7, more preferably about 5.5 to about 6.5.

In some embodiments, the aforementioned pharmaceutical composition comprises: (a) about 90 mg/mL to about 150 mg/mL of an anti-TSLP antibody, (b) about 10 mM to about 40 mM of a sodium phosphate buffering agent, a histidine buffering agent or a sodium acetate buffering agent, (c) about 0.008% (w/v) to about 0.04% (w/v) of polysorbate 80, and (d) about 150 mM to about 600 mM of mannitol, and/or about 150 mM to about 600 mM of arginine or a pharmaceutically acceptable salt thereof, or proline; wherein the pharmaceutical composition has a pH of about 5 to about 7, preferably about 5.5 to about 7, more preferably about 5.5 to about 6.5.

In some embodiments, the aforementioned pharmaceutical composition comprises: (a) about 120 mg/mL to about 150 mg/mL of an anti-TSLP antibody, (b) about 10 mM to about 40 mM of a sodium phosphate buffering agent, a histidine buffering agent, or a sodium acetate buffering agent, (c) about 0.008% (w/v) to about 0.04% (w/v) of polysorbate 80, and (d) about 150 mM to about 600 mM of mannitol, and/or about 150 mM to about 600 mM of arginine or a pharmaceutically acceptable salt thereof, or proline; wherein the pharmaceutical composition has a pH of about 5 to about 7, preferably about 5.5 to about 7, more preferably about 5.5 to about 6.5.

In some embodiments, the aforementioned pharmaceutical composition comprises: (a) about 120 mg/mL to about 150 mg/mL of an anti-TSLP antibody, (b) about 10 mM to about 40 mM of a histidine buffering agent, (c) about 0.01% (w/v) to about 0.04% (w/v) of polysorbate 80 or polysorbate 20, and (d) about 150 mM to about 600 mM of proline, mannitol or arginine; wherein the pharmaceutical composition has a pH of about 5.5 to about 6.5, preferably about 5.5 to about 6, e.g., about 5.5, about 5.6, about 5.7, about 5.8, about 5.9, or about 6.

In some embodiments, the aforementioned pharmaceutical composition comprises: (a) about 120 mg/mL to about 150 mg/mL of an anti-TSLP antibody, (b) about 20 mM of a histidine buffering agent, (c) about 0.02% (w/v) of polysorbate 80, and (d) about 200 mM of mannitol; wherein the pharmaceutical composition has a pH of about 5.5 to about 6.5, preferably about 5.5 to about 6.

In some embodiments, the aforementioned pharmaceutical composition comprises: (a) about 120 mg/mL to about 150 mg/mL of an anti-TSLP antibody, (b) about 20 mM of a histidine buffering agent, (c) about 0.02% (w/v) of polysorbate 80, and (d) about 150 mM of arginine; wherein the pharmaceutical composition has a pH of about 5.5 to about 6.5, preferably about 5.5 to about 6.

In some embodiments, the aforementioned pharmaceutical composition comprises: (a) about 120 mg/mL to about 150 mg/mL of an anti-TSLP antibody, (b) about 20 mM of a histidine buffering agent, (c) about 0.02% (w/v) of polysorbate 80, and (d) about 400 mM of proline; wherein the pharmaceutical composition has a pH of about 5.5 to about 6.5, preferably about 5.5 to about 6.

In some embodiments, the aforementioned pharmaceutical composition comprises: (a) about 120 mg/mL to about 150 mg/mL of an anti-TSLP antibody, (b) about 18 mM of a histidine buffering agent, (c) about 0.02% (w/v) of polysorbate 80, and (d) about 400 mM of proline; wherein the pharmaceutical composition has a pH of about 5.5 to about 6.5, preferably about 5.5 to about 6.

In some embodiments, the aforementioned pharmaceutical composition comprises: (a) about 120 mg/mL to about 150 mg/mL of an anti-TSLP antibody, (b) about 22 mM of a histidine buffering agent, (c) about 0.02% (w/v) of polysorbate 80, and (d) about 400 mM of proline; wherein the pharmaceutical composition has a pH of about 5.5 to about 6.5, preferably about 5.5 to about 6.

In some embodiments, the aforementioned pharmaceutical composition comprises: (a) about 120 mg/mL to about 150 mg/mL of an anti-TSLP antibody, (b) about 18 mM of a histidine buffering agent, (c) about 0.02% (w/v) of polysorbate 80, and (d) about 500 mM of proline; wherein the pharmaceutical composition has a pH of about 5.5 to about 6.5, preferably about 5.5 to about 6.

In some embodiments, the aforementioned pharmaceutical composition comprises: (a) about 120 mg/mL to about 150 mg/mL of an anti-TSLP antibody, (b) about 22 mM of a histidine buffering agent, (c) about 0.02% (w/v) of polysorbate 80, and (d) about 500 mM of proline; wherein the pharmaceutical composition has a pH of about 5.5 to about 6.5, preferably about 5.5 to about 6.

In some embodiments, the aforementioned pharmaceutical composition comprises: (a) about 120 mg/mL to about 150 mg/mL of an anti-TSLP antibody, (b) about 20 mM of a histidine buffering agent, (c) about 0.02% (w/v) of polysorbate 80, and (d) about 500 mM of proline; wherein the pharmaceutical composition has a pH of about 5.5 to about 6.5, preferably about 5.5 to about 6.

In some embodiments, the aforementioned pharmaceutical composition comprises: (a) about 120 mg/mL to about 150 mg/mL of an anti-TSLP antibody, (b) about 18 mM of a histidine buffering agent, (c) about 0.02% (w/v) of polysorbate 80, and (d) about 450 mM of proline; wherein the pharmaceutical composition has a pH of about 5.5 to about 6.5, preferably about 5.5 to about 6.

In some embodiments, the aforementioned pharmaceutical composition comprises: (a) about 120 mg/mL to about 150 mg/mL of an anti-TSLP antibody, (b) about 22 mM of a histidine buffering agent, (c) about 0.02% (w/v) of polysorbate 80, and (d) about 450 mM of proline; wherein the pharmaceutical composition has a pH of about 5.5 to about 6.5, preferably about 5.5 to about 6.

In some embodiments, the aforementioned pharmaceutical composition comprises: (a) about 120 mg/mL to about 150 mg/mL of an anti-TSLP antibody, (b) about 20 mM of a histidine buffering agent, (c) about 0.02% (w/v) of polysorbate 80, and (d) about 450 mM of proline; wherein the pharmaceutical composition has a pH of about 5.5 to about 6.5, preferably about 5.5 to about 6.

In some embodiments, the aforementioned pharmaceutical composition comprises: (a) about 120 mg/mL to about 150 mg/mL of an anti-TSLP antibody, (b) about 18 mM of a histidine buffering agent, (c) about 0.02% (w/v) of polysorbate 80, and (d) about 350 mM of proline; wherein the pharmaceutical composition has a pH of about 5.5 to about 6.5, preferably about 5.5 to about 6.

In some embodiments, the aforementioned pharmaceutical composition comprises: (a) about 120 mg/mL to about 150 mg/mL of an anti-TSLP antibody, (b) about 22 mM of a histidine buffering agent, (c) about 0.02% (w/v) of polysorbate 80, and (d) about 350 mM of proline; wherein the pharmaceutical composition has a pH of about 5.5 to about 6.5, preferably about 5.5 to about 6.

In some embodiments, the aforementioned pharmaceutical composition comprises: (a) about 120 mg/mL to about 150 mg/mL of an anti-TSLP antibody, (b) about 20 mM of a histidine buffering agent, (c) about 0.02% (w/v) of polysorbate 80, and (d) about 350 mM of proline; wherein the pharmaceutical composition has a pH of about 5.5 to about 6.5, preferably about 5.5 to about 6.

In some embodiments, the aforementioned pharmaceutical composition comprises: (a) about 120 mg/mL to about 150 mg/mL of an anti-TSLP antibody, (b) about 18 mM of a histidine buffering agent, (c) about 0.02% (w/v) of polysorbate 80, and (d) about 250 mM of proline; wherein the pharmaceutical composition has a pH of about 5.5 to about 6.5, preferably about 5.5 to about 6.

In some embodiments, the aforementioned pharmaceutical composition comprises: (a) about 120 mg/mL to about 150 mg/mL of an anti-TSLP antibody, (b) about 22 mM of a histidine buffering agent, (c) about 0.02% (w/v) of polysorbate 80, and (d) about 250 mM of proline; wherein the pharmaceutical composition has a pH of about 5.5 to about 6.5, preferably about 5.5 to about 6.

In some embodiments, the aforementioned pharmaceutical composition comprises: (a) about 120 mg/mL to about 150 mg/mL of an anti-TSLP antibody, (b) about 20 mM of a histidine buffering agent, (c) about 0.02% (w/v) of polysorbate 80, and (d) about 250 mM of proline; wherein the pharmaceutical composition has a pH of about 5.5 to about 6.5, preferably about 5.5 to about 6.

In some embodiments, the aforementioned pharmaceutical composition comprises: (a) about 120 mg/mL of an anti-TSLP antibody, (b) about 20 mM of a histidine buffering agent, (c) about 0.02% (w/v) of polysorbate 80, and (d) about 400 mM of proline; wherein the pharmaceutical composition has a pH of about 5.8.

In some embodiments, the aforementioned pharmaceutical composition comprises: (a) about 120 mg/mL to about 150 mg/mL of an anti-TSLP antibody, (b) about 10 mM to about 40 mM of a phosphate or acetate buffering agent, (c) about 0.01% (w/v) to about 0.04% (w/v) of polysorbate 80 or polysorbate 20, and (d) about 200 mM to about 600 mM of proline, arginine or mannitol; wherein the pharmaceutical composition has a pH of about 5 to about 7, e.g., about 5, about 5.1, about 5.2, about 5.3, about 5.4, about 5.5, about 5.6, about 5.7, about 5.8, about 5.9, about 6, about 6.5, or about 7.

In some embodiments, the aforementioned pharmaceutical composition comprises: (a) about 120 mg/mL to about 150 mg/mL of an anti-TSLP antibody, (b) about 20 mM of a phosphate or acetate buffering agent, (c) about 0.01% (w/v) to about 0.04% (w/v) of polysorbate 80 or polysorbate 20, and (d) about 200 mM of mannitol; wherein the pharmaceutical composition has a pH of about 5 to about 7.

In some embodiments, the aforementioned pharmaceutical composition comprises: (a) about 120 mg/mL to about 150 mg/mL of an anti-TSLP antibody, (b) about 20 mM of a sodium phosphate or sodium acetate buffering agent, (c) about 0.02% (w/v) to about 0.04% (w/v) of polysorbate 80 or polysorbate 20, and (d) about 200 mM of mannitol; wherein the pharmaceutical composition has a pH of about 5 to about 7.

In the aforementioned pharmaceutical composition, an isotonizing agent or a preservative may be appropriately added as desired, and the isotonizing agent or the preservative may be appropriately used in an appropriate amount within a range of an amount capable of achieving a desired effect. The isotonizing agent comprises sodium chloride, potassium chloride, calcium chloride, etc.; the preservative comprises methyl p-hydroxybenzoate, ethyl p-hydroxybenzoate, sorbic acid, phenol, cresol, chlorocresol, benzyl alcohol, etc.

In an optional embodiment, the anti-TSLP antibody in the aforementioned pharmaceutical composition comprises a heavy chain variable region comprising a VH-CDR1 region, a VH-CDR2 region, and a VH-CDR3 region, wherein the VH-CDR1 region, the VH-CDR2 region, and the VH-CDR3 region comprise: amino acid sequences set forth in SEQ ID NOs: 1, 2 and 3, respectively.

In an optional embodiment, the anti-TSLP antibody in the aforementioned pharmaceutical composition comprises a heavy chain variable region comprising an amino acid sequence set forth in SEQ ID NO: 7, 8 or 9 (X1 = R, X2 = V, X3 = R; X1 = R, X2 = V, X3 = V; X1 = R, X2 = A, X3 = R; X1 = K, X2 = A, X3 = R; or X1 = K, X2 = A, X3 = V); wherein, the amino acid sequence set forth in SEQ ID NO: 7 can be encoded by a nucleotide sequence set forth in SEQ ID NO: 17 or 18, and the amino acid sequence set forth in SEQ ID NO: 9 (X1 = R, X2 = V, X3 = R) can be encoded by a nucleotide sequence set forth in SEQ ID NO: 19.

In an optional embodiment, the anti-TSLP antibody in the aforementioned pharmaceutical composition comprises a light chain variable region comprising a VL-CDR1 region, a VL-CDR2 region, and a VL-CDR3 region, wherein the VL-CDR1 region, the VL-CDR2 region, and the VL-CDR3 region may comprise: amino acid sequences set forth in SEQ ID NOs: 4, 5 and 6, respectively.

In an optional embodiment, the anti-TSLP antibody in the aforementioned pharmaceutical composition comprises a light chain variable region comprising an amino acid sequence set forth in SEQ ID NO: 10 or 11 (X1 = S, X2 = V; X1 = A, X2 = I; or X1 = S, X2 = I); wherein, the amino acid sequence set forth in SEQ ID NO: 10 can be encoded by a nucleotide sequence set forth in SEQ ID NO: 20 or 21, and the amino acid sequence set forth in SEQ ID NO: 11 (X1 = A, X2 = I) can be encoded by a nucleotide sequence set forth in SEQ ID NO: 22.

In an optional embodiment, the anti-TSLP antibody in the aforementioned pharmaceutical composition comprises a heavy chain variable region comprising a VH-CDR1 region, a VH-CDR2 region and a VH-CDR3 region, and a light chain variable region comprising a VL-CDR1 region, a VL-CDR2 region and a VL-CDR3 region, wherein the VH-CDR1 region, the VH-CDR2 region, the VH-CDR3 region, the VL-CDR1 region, the VL-CDR2 region, and the VL-CDR3 region comprise: amino acid sequences set forth in SEQ ID NOs: 1, 2, 3, 4, 5 and 6, respectively.

In an optional embodiment, the anti-TSLP antibody in the aforementioned pharmaceutical composition comprises a heavy chain variable region and a light chain variable region, and the heavy chain variable region and the light chain variable region may comprise an amino acid sequence selected from the following amino acid sequences: (1) respectively as set forth in SEQ ID NOs: 7 and 10; (2) respectively as set forth in SEQ ID NOs: 8 and 11 (X1 = S, X2 = V); (3) respectively as set forth in SEQ ID NOs: 9 (X1 = R, X2 = V, X3 = R) and 11 (X1 = S, X2 = V); (4) respectively as set forth in SEQ ID NOs: 9 (X1 = R, X2 = V, X3 = V) and 11 (X1 = S, X2 = V); (5) respectively as set forth in SEQ ID NOs: 9 (X1 = R, X2 = A, X3 = R) and 11 (X1 = S, X2 = V); (6) respectively as set forth in SEQ ID NOs: 9 (X1 = K, X2 = A, X3 = R) and 11 (X1 = S, X2 = V); (7) respectively as set forth in SEQ ID NOs: 9 (X1 = K, X2 = A, X3 = V) and 11 (X1 = S, X2 = V); (8) respectively as set forth in SEQ ID NOs: 8 and 11 (X1 = A, X2 = I); (9) respectively as set forth in SEQ ID NOs: 9 (X1 = R, X2 = V, X3 = R) and 11 (X1 = A, X2 = I); (10) respectively as set forth in SEQ ID NOs: 9 (X1 = R, X2 = V, X3 = V) and 11 (X1 = A, X2 = I); (11) respectively as set forth in SEQ ID NOs: 9 (X1 = R, X2 = A, X3 = R) and 11 (X1 = A, X2 = I); (12) respectively as set forth in SEQ ID NOs: 9 (X1 = K, X2 = A, X3 = R) and 11 (X1 = A, X2 = I); (13) respectively as set forth in SEQ ID NOs: 9 (X1 = K, X2 = A, X3 = V) and 11 (X1 = A, X2 = I); (14) respectively as set forth in SEQ ID NOs: 8 and 11 (X1 = S, X2 = I); (15) respectively as set forth in SEQ ID NOs: 9 (X1 = R, X2 = V, X3 = R) and 11 (X1 = S, X2 = I); (16) respectively as set forth in SEQ ID NOs: 9 (X1 = R, X2 = V, X3 = V) and 11 (X1 = S, X2 = I); (17) respectively as set forth in SEQ ID NOs: 9 (X1 = R, X2 = A, X3 = R) and 11 (X1 = S, X2 = I); (18) respectively as set forth in SEQ ID NOs: 9 (X1 = K, X2 = A, X3 = R) and 11 (X1 = S, X2 = I); or (19) respectively as set forth in SEQ ID NOs: 9 (X1 = K, X2 = A, X3 = V) and 11 (X1 = S, X2 = I).

In an optional embodiment, the anti-TSLP antibody in the aforementioned pharmaceutical composition comprises a heavy chain and a light chain, the heavy chain comprises a heavy chain variable region and a heavy chain constant region, and the light chain comprises a light chain variable region and a light chain constant region; the heavy chain constant region may comprise a human IgG1 heavy chain constant region having an amino acid sequence set forth in SEQ ID NO: 12 or 37, or a human IgG4 heavy chain constant region having an amino acid sequence set forth in SEQ ID NO: 13, or a fragment of the heavy chain constant region; the light chain constant region may comprise a human κ light chain constant region having an amino acid sequence set forth in SEQ ID NO: 14 or a fragment thereof; the heavy chain constant region may also be a mouse IgG1 heavy chain constant region having an amino acid sequence set forth in SEQ ID NO: 15, and the light chain constant region may be a mouse κ light chain constant region having an amino acid sequence set forth in SEQ ID NO: 16; wherein, the amino acid sequences set forth in SEQ ID NOs: 12-16 and 37 may be encoded by the nucleotide sequences set forth in SEQ ID NOs: 23-27 and 38, respectively.

In an optional embodiment, the anti-TSLP antibody in the aforementioned pharmaceutical composition may be a full-length antibody, e.g., of the IgG1, IgG2 or IgG4 isotype. In other embodiments, the anti-TSLP antibody in the aforementioned pharmaceutical composition may be a single chain variable (scFv) antibody, or an antibody fragment, such as a Fab or F(ab')₂ fragment.

In an optional embodiment, the anti-TSLP antibody in the aforementioned pharmaceutical composition is an anti-TSLP antibody in PCT/CN2020/113289.

The present disclosure also provides a method for preparing the aforementioned pharmaceutical composition, comprising the step of contacting the above-mentioned anti-TSLP antibody with a buffering agent, for example, displacing the anti-TSLP antibody into the buffering agent, preferably, the buffering agent is a phosphate buffering agent, a histidine buffering agent or an acetate buffering agent, the buffering agent has a concentration of 1 mM to 100 mM, or 2 mM to 80 mM, preferably 5 mM to 60 mM, more preferably 10 mM to 40 mM, and the buffering agent has a pH of 5 to 7, preferably 5.5 to 7, more preferably 5.5 to 6.5. The method for preparing the aforementioned pharmaceutical composition further comprises adding one or more stabilizers and a surfactant in any order, wherein the stabilizer comprises trehalose, mannitol, sucrose, arginine or a pharmaceutically acceptable salt thereof, glycine or proline, etc., and the surfactant comprises polysorbate 20 or polysorbate 80, etc.

The present disclosure also provides a method for preparing a lyophilized formulation comprising an anti-TSLP antibody, comprising the step of lyophilizing the aforementioned pharmaceutical composition. In some embodiments, the lyophilization is carried out using a method well known in the art, and comprises, but is not limited to, steps of pre-freezing, primary drying and secondary drying. It is understood by those skilled in the art that any method for removing water from the pharmaceutical composition of the present disclosure is suitable for use in the present disclosure. The present disclosure also provides a lyophilized formulation comprising an anti-TSLP antibody, which is prepared by the aforementioned method for preparing a lyophilized formulation.

The present disclosure also provides a lyophilized formulation comprising an anti-TSLP antibody, the lyophilized formulation being capable of forming the aforementioned pharmaceutical composition upon reconstitution.

The present disclosure also provides an article of manufacture comprising a container comprising the aforementioned pharmaceutical composition or the aforementioned lyophilized formulation.

The pharmaceutical composition or the lyophilized formulation of the present disclosure may be administered according to known methods, e.g., by injection or infusion over a period of time in a suitable manner, e.g., by subcutaneous, intravenous, intraperitoneal, intramuscular, intraarterial, intralesional or intraarticular routes, topically, by inhalation or by sustained or delayed release.

The pharmaceutical composition of the present disclosure may also comprise one or more other active compounds, preferably those having complementary activities, which do not adversely affect each other, as long as necessary for the particular condition in need of being treated. Additionally or alternatively, the pharmaceutical composition may comprise an anti-TSLP antibody and another disease-specific protein, such as TSLPR, IgE, IL-13, or IL-5, such molecules being suitably present in the pharmaceutical composition in an amount effective for the intended target.

The present disclosure also provides use of the pharmaceutical composition, the lyophilized formulation, or the article of manufacture of the present disclosure for preparing a medicament for treating and preventing TSLP-related diseases. In some embodiments, the TSLP-related diseases include asthma, ulcerative colitis, atopic dermatitis, and psoriasis.

The present disclosure also provides a method for treating and preventing TSLP-related diseases, comprising administering to a subject the pharmaceutical composition, the lyophilized formulation, or the article of manufacture of the present disclosure. In some embodiments, the TSLP-related diseases include asthma, ulcerative colitis, atopic dermatitis, and psoriasis.

In general, the present disclosure relates to the following items:
1. A pharmaceutical composition, comprising: (a) an anti-TSLP antibody, (b) a buffering agent, (c) a surfactant, and one or more (d) stabilizers.
2. The pharmaceutical composition according to item 1, wherein the anti-TSLP antibody comprises a heavy chain variable region comprising a VH-CDR1 region, a VH-CDR2 region and a VH-CDR3 region, and a light chain variable region comprising a VL-CDR1 region, a VL-CDR2 region and a VL-CDR3 region, wherein the VH-CDR1 region, the VH-CDR2 region, the VH-CDR3 region, the VL-CDR1 region, the VL-CDR2 region, and the VL-CDR3 region comprise: amino acid sequences set forth in SEQ ID NOs: 1, 2, 3, 4, 5 and 6, respectively.
3. The pharmaceutical composition according to item 1 or 2, wherein the anti-TSLP antibody comprises a heavy chain variable region and a light chain variable region, and the heavy chain variable region and the light chain variable region may comprise an amino acid sequence selected from the following amino acid sequences: (1) respectively as set forth in SEQ ID NOs: 7 and 10; (2) respectively as set forth in SEQ ID NOs: 8 and 11 (X1 = S, X2 = V); (3) respectively as set forth in SEQ ID NOs: 9 (X1 = R, X2 = V, X3 = R) and 11 (X1 = S, X2 = V); (4) respectively as set forth in SEQ ID NOs: 9 (X1 = R, X2 = V, X3 = V) and 11 (X1 = S, X2 = V); (5) respectively as set forth in SEQ ID NOs: 9 (X1 = R, X2 = A, X3 = R) and 11 (X1 = S, X2 = V); (6) respectively as set forth in SEQ ID NOs: 9 (X1 = K, X2 = A, X3 = R) and 11 (X1 = S, X2 = V); (7) respectively as set forth in SEQ ID NOs: 9 (X1 = K, X2 = A, X3 = V) and 11 (X1 = S, X2 = V); (8) respectively as set forth in SEQ ID NOs: 8 and 11 (X1 = A, X2 = I); (9) respectively as set forth in SEQ ID NOs: 9 (X1 = R, X2 = V, X3 = R) and 11 (X1 = A, X2 = I); (10) respectively as set forth in SEQ ID NOs: 9 (X1 = R, X2 = V, X3 = V) and 11 (X1 = A, X2 = I); (11) respectively as set forth in SEQ ID NOs: 9 (X1 = R, X2 = A, X3 = R) and 11 (X1 = A, X2 = I); (12) respectively as set forth in SEQ ID NOs: 9 (X1 = K, X2 = A, X3 = R) and 11 (X1 = A, X2 = I); (13) respectively as set forth in SEQ ID NOs: 9 (X1 = K, X2 = A, X3 = V) and 11 (X1 = A, X2 = I); (14) respectively as set forth in SEQ ID NOs: 8 and 11 (X1 = S, X2 = I); (15) respectively as set forth in SEQ ID NOs: 9 (X1 = R, X2 = V, X3 = R) and 11 (X1 = S, X2 = I); (16) respectively as set forth in SEQ ID NOs: 9 (X1 = R, X2 = V, X3 = V) and 11 (X1 = S, X2 = I); (17) respectively as set forth in SEQ ID NOs: 9 (X1 = R, X2 = A, X3 = R) and 11 (X1 = S, X2 = I); (18) respectively as set forth in SEQ ID NOs: 9 (X1 = K, X2 = A, X3 = R) and 11 (X1 = S, X2 = I); or (19) respectively as set forth in SEQ ID NOs: 9 (X1 = K, X2 = A, X3 = V) and 11 (X1 = S, X2 = I).
4. The pharmaceutical composition according to any one of items 1-3, wherein the anti-TSLP antibody has a concentration of 30 mg/mL to 300 mg/mL, preferably 50 mg/mL to 250 mg/mL, preferably 70 mg/mL to 200 mg/mL, more preferably 90 mg/mL to 150 mg/mL, most preferably 120 mg/mL to 150 mg/mL.
5. The pharmaceutical composition according to any one of items 1-4, wherein the buffering agent comprises a phosphate buffering agent, a histidine buffering agent, an acetate buffering agent, or a citrate buffering agent.
6. The pharmaceutical composition according to item 5, wherein the phosphate buffering agent is a sodium phosphate buffering agent, the acetate buffering agent is a sodium acetate buffering agent, and the citrate buffering agent is a sodium citrate buffering agent.
7. The pharmaceutical composition according to any one of items 1-6, wherein the buffering agent has a concentration of 1 mM to 100 mM, preferably 2 mM to 80 mM, more preferably 5 mM to 60 mM, most preferably 10 mM to 40 mM.
8. The pharmaceutical composition according to any one of items 1-7, wherein the surfactant comprises polysorbate 80 or polysorbate 20.
9. The pharmaceutical composition according to any one of items 1-8, wherein the surfactant has a concentration of 0.001% (w/v) to 0.1% (w/v), preferably 0.004% (w/v) to 0.08% (w/v), more preferably 0.006% (w/v) to 0.06% (w/v), most preferably 0.008% (w/v) to 0.04% (w/v).
10. The pharmaceutical composition according to any one of items 1-9, wherein the stabilizer comprises trehalose, mannitol, sucrose, arginine or a pharmaceutically acceptable salt thereof, glycine, or proline.
11. The pharmaceutical composition according to any one of items 1-10, wherein the stabilizer has a concentration of 100 mM to 1000 mM, preferably 120 mM to 800 mM, more preferably 150 mM to 700 mM, most preferably 150 mM to 600 mM.
12. The pharmaceutical composition according to any one of items 1-11, wherein the pharmaceutical composition has a pH of 5 to 7, preferably 5.5 to 7, more preferably 5.5 to 6.5.
13. The pharmaceutical composition according to any one of items 1-12, wherein the pharmaceutical composition comprises:
   (a) 30 mg/mL to 300 mg/mL of an anti-TSLP antibody,
   (b) 1 mM to 100 mM of a buffering agent,
   (c) 0.001% (w/v) to 0.1% (w/v) of a surfactant, and

   one or more (d) 100 mM to 1000 mM of stabilizers;
   wherein the pharmaceutical composition has a pH of 5 to 7, preferably 5.5 to 7, more preferably 5.5 to 6.5.
14. The pharmaceutical composition according to any one of items 1-13, wherein the pharmaceutical composition comprises:
   (a) 50 mg/mL to 250 mg/mL of an anti-TSLP antibody,
   (b) 2 mM to 80 mM of a buffering agent,
   (c) 0.004% (w/v) to 0.08% (w/v) of a surfactant, and

   one or more (d) 120 mM to 800 mM of stabilizers;
   wherein the pharmaceutical composition has a pH of 5 to 7, preferably 5.5 to 7, more preferably 5.5 to 6.5.
15. The pharmaceutical composition according to any one of items 1-14, wherein the pharmaceutical composition comprises:
   (a) 70 mg/mL to 200 mg/mL of an anti-TSLP antibody,
   (b) 5 mM to 60 mM of a buffering agent,
   (c) 0.006% (w/v) to 0.06% (w/v) of a surfactant, and

   one or more (d) 150 mM to 700 mM of stabilizers;
   wherein the pharmaceutical composition has a pH of 5 to 7, preferably 5.5 to 7, more preferably 5.5 to 6.5.
16. The pharmaceutical composition according to any one of items 1-15, wherein the pharmaceutical composition comprises:
   (a) 90 mg/mL to 150 mg/mL of an anti-TSLP antibody,
   (b) 10 mM to 40 mM of a buffering agent,
   (c) 0.008% (w/v) to 0.04% (w/v) of a surfactant, and

   one or more (d) 150 mM to 600 mM of stabilizers;
   wherein the pharmaceutical composition has a pH of 5 to 7, preferably 5.5 to 7, more preferably 5.5 to 6.5.
17. The pharmaceutical composition according to any one of items 1-16, wherein the pharmaceutical composition comprises:
   (A) 120 mg/mL to 150 mg/mL of an anti-TSLP antibody,
   (b) 10 mM to 40 mM of a buffering agent,
   (c) 0.008% (w/v) to 0.04% (w/v) of a surfactant, and
   one or more (d) 150 mM to 600 mM of stabilizers;
   wherein the pharmaceutical composition has a pH of 5 to 7, preferably 5.5 to 7, more preferably 5.5 to 6.5.
18. A lyophilized formulation comprising an anti-TSLP antibody, the lyophilized formulation being obtained by lyophilizing the pharmaceutical composition according to any one of items 1-17.
19. A lyophilized formulation comprising an anti-TSLP antibody, the lyophilized formulation being capable of forming the pharmaceutical composition according to any one of items 1-17 upon reconstitution.
20. An article of manufacture, comprising a container comprising the pharmaceutical composition according to any one of items 1-17, or the lyophilized formulation according to any one of items 18-19.
21. A method for treating and preventing TSLP-related diseases, comprising administering to a patient in need thereof a therapeutically effective amount of the pharmaceutical composition according to any one of items 1-17 or the lyophilized formulation according to any one of items 18-19 or the article of manufacture according to item 20.
22. The method according to item 21, wherein the TSLP-related diseases include asthma, ulcerative colitis, atopic dermatitis, and psoriasis.

### BRIEF DESCRIPTION OF THE DRAWINGS

FIG. 1 shows the binding ability of humanized antibodies hu1C5F12E9-V8 and hu1C5F12E9-V14 to human TSLP in a capture ELISA.
FIG. 2 shows the binding ability of humanized antibodies hu1C5F12E9-V8 and hu1C5F12E9-V14 to cynomolgus monkey TSLP in an indirect ELISA.
FIG. 3 shows the ability of humanized antibodies hu1C5F12E9-V8 and hu1C5F12E9-V14 to block the binding of human TSLP to TSLPR/IL7R in a competitive ELISA.
FIG. 4 shows the ability of humanized antibodies hu1C5F12E9-V8 and hu1C5F12E9-V14 to block the binding of the benchmark Tezepelumab to human TSLP in a competitive ELISA.
FIG. 5 shows the ability of humanized antibodies hu1C5F12E9-V8 and hu1C5F12E9-V14 to block the binding of human TSLP to engineered BAF3 cells expressing human TSLPR and IL7R in a cell-based ligand blocking FACS assay.
FIGs. 6A-6B show the inhibitory effect of humanized antibodies hu1C5F12E9-V8 (A) and hu1C5F12E9-V14 (B) against the survival and proliferation of BAF3 cells in a cell-based functional assay.
FIG. 7 shows the binding ability of humanized antibodies hu1C5F12E9-V8 (IgG1), hu1C5F12E9-V8 (IgG4), hu1C5F12E9-V14 (IgG1) and hu1C5F12E9-V14 (IgG4) to human TSLP in a capture ELISA.
FIG. 8 shows the ability of humanized antibodies hu1C5F12E9-V8 (IgG1), hu1C5F12E9-V8 (IgG4), hu1C5F12E9-V14 (IgG1) and hu1C5F12E9-V14 (IgG4) to block the binding of human TSLP to TSLPR/IL7R in a competitive ELISA.
FIG. 9 shows the ability of humanized antibodies hu1C5F12E9-V8 (IgG1), hu1C5F12E9-V8 (IgG4), hu1C5F12E9-V14 (IgG1) and hu1C5F12E9-V14 (IgG4) to block the binding of the benchmark to human TSLP in a competitive ELISA.
FIG. 10 shows the ability of humanized antibodies hu1C5F12E9-V8 (IgG1), hu1C5F12E9-V8 (IgG4), hu1C5F12E9-V14 (IgG1) and hu1C5F12E9-V14 (IgG4) to block the binding of human TSLP to engineered BAF3 cells expressing human TSLPR and IL7R in a cell-based ligand blocking FACS assay.
FIGs. 11A-11B show the inhibitory effect of humanized antibodies hu1C5F12E9-V8 (IgG1), hu1C5F12E9-V8 (IgG4) (A), hu1C5F12E9-V14 (IgG1) and hu1C5F12E9-V14 (IgG4) (B) against the survival and proliferation of BAF3 cells in a cell-based functional assay.
FIGs. 12A-12B show the ability of humanized antibodies hu1C5F12E9-V8 (IgG1), hu1C5F12E9-V8 (IgG4) (A), hu1C5F12E9-V14 (IgG1) and hu1C5F12E9-V14 (IgG4) (B) to block the interaction between human TSLP and engineered HEK293T cells in a cell-based reporter gene assay.
FIG. 13 shows the result of protein thermal shift assay for antibodies hu1C5F12E9-V8 (IgG1), hu1C5F12E9-V8 (IgG4), hu1C5F12E9-V14 (IgG1) and hu1C5F12E9-V14 (IgG4).

### DETAILED DESCRIPTION

The following description of the present disclosure is intended to be only illustrative of various embodiments of the present disclosure.

In order to better understand the present disclosure, certain technical and scientific terms are specifically defined below. Unless otherwise specifically defined herein, all other technical and scientific terms used herein have the meanings which are commonly understood by those of ordinary skill in the art to which the present disclosure belongs.

The "phosphate buffering agent" is a buffering agent comprising phosphate ions. Examples of the phosphate buffering agent include a sodium phosphate buffering agent, a potassium phosphate buffering agent, etc., and the preferred phosphate buffering agent is a sodium phosphate buffering agent.

The "acetate buffering agent" is a buffering agent comprising acetate ions. Examples of the acetate buffering agent include a potassium acetate buffering agent, an ammonium acetate buffering agent, a sodium acetate buffering agent, etc., and the preferred acetate buffering agent is a sodium acetate buffering agent.

The "citrate buffering agent" is a buffering agent comprising citrate ions. Examples of the citrate buffering agent include a sodium citrate buffering agent, a potassium citrate buffering agent, a calcium citrate buffering agent, etc., and the preferred citrate buffering agent is a sodium citrate buffering agent.

The "histidine buffering agent" is a buffering agent comprising histidine ions. Examples of the histidine buffering agent include a histidine-acetic acid buffering agent, a histidine-hydrochloric acid buffering agent, and a histidine-histidine hydrochloride buffering agent. Preferably, the histidine buffering agent is prepared by L-histidine, and pH is further adjusted with acetic acid or hydrochloric acid.

The "buffering agent" refers to a pharmaceutically acceptable agent capable of maintaining the pH of the pharmaceutical composition to the desired pH range. Buffering agents suitable for use in the present disclosure include a phosphate buffering agent, an acetate buffering agent, a citrate buffering agent, or a histidine buffering agent. In a preferred embodiment, the buffering agent suitable for use in the present disclosure is a histidine buffering agent, prepared by L-histidine, and the pH is further adjusted with acetic acid or hydrochloric acid.

The "stabilizer" refers to a pharmaceutically acceptable agent used to maintain the stability of active ingredients in the pharmaceutical composition. In the present disclosure, the stabilizer also functions as an anti-sticking agent and/or an isotonizing agent.

The "pharmaceutical composition" is meant to encompass a product comprising a particular active ingredient (e.g., an antibody), optionally in a particular amount, as well as any product which results, directly or indirectly, from combining the particular active ingredients, optionally in the particular amount. The purpose of the pharmaceutical composition is to make the antibody suitable for production and administration to patients and to maintain biological activity and/or stability during storage and subsequent use. In some embodiments, the pharmaceutical composition is a water-soluble injection, including but not limited to a water-soluble formulation not lyophilized or a water-soluble formulation obtained by reconstituting a lyophilized powder. In other embodiments, the pharmaceutical composition is a lyophilized formulation. In the present disclosure, the "pharmaceutical composition" and the "formulation" are not mutually exclusive.

"Stable" or "stabilized" pharmaceutical compositions are those in which an active ingredient (e.g., an antibody) substantially retains its physical and/or chemical stability and/or biological activity during storage. Various analytical techniques for determining the stability of an active ingredient are known in the art, for example, reviewed in Peptide and Protein Drug Delivery, 247-301, Vincent Lee Ed., Marcel Dekker, Inc., New York, N.Y, Pubs. (1991) and Jones, A. Adv. Drug Delivery Rev. 10:29-90 (1993). Stability can be measured at selected temperatures and under other storage conditions over a selected period of time. For example, an active ingredient "retains its physical stability" in a pharmaceutical composition if it does not exhibit a significant increase in aggregation, precipitation and/or denaturation upon visual inspection of color and/or clarity, or as measured by UV light scattering, size exclusion chromatography (SEC) and differential scanning calorimetry (DSC). Preferably, when the pharmaceutical composition of the present disclosure is used, 5% or less, 4% or less, preferably 3% or less of the active ingredient forms aggregates (also called high-molecular impurities), as measured by, for example, SEC-UPLC or any other suitable method for measuring aggregate formation. An active ingredient (e.g., an antibody) "retains its chemical stability" in a pharmaceutical composition if the active ingredient does not exhibit a significant chemical change. Chemical stability can be assessed by detecting and quantifying the chemically altered formats of the antibody. The processes that often alter the chemical structure of a protein include hydrolysis or truncation (assessed by methods such as size exclusion chromatography and SDS-PAGE), oxidation (assessed by methods such as peptide mapping coupled with mass spectrometry or MALDI/TOF/MS), deamidation (assessed by methods such as ion exchange chromatography, capillary isoelectric focusing, peptide mapping and isoaspartic acid measurement) and isomerization (assessed by measuring isoaspartic acid content, by peptide mapping, etc.). An active ingredient (e.g., an antibody) "retains its biological activity" for a given time in a pharmaceutical composition, as determined, for example, by an antigen binding assay, if the biological activity of the active ingredient for the given time is within a predetermined range of the biological activity exhibited when the pharmaceutical composition is prepared. Other methods for assessing the stability of a pharmaceutical composition are also described in the examples below, such as measuring viscosity using an HVROC-S viscometer. Preferably, the pharmaceutical composition of the present disclosure is considered to have a low viscosity when it exhibits a viscosity of about 20 mpa•s, about 19 mpa•s, about 18 mpa•s, about 15 mpa•s, or less. The "high-molecular impurities" or "aggregates" refer to the generic term for impurities having a molecular weight greater than that of the active ingredient of interest (e.g., an antibody).

The "charge variants" refers to variants of an antibody that undergo glycosylation, deamidation, oxidation, and/or isomerization, etc., and that directly or indirectly cause changes in the charges of the antibody molecules, and these charge variants can be detected by capillary isoelectric focusing electrophoresis (CIEF) and cation exchange chromatography (CEX-HPLC), etc.

The articles "a", "an" and "the" are used herein to refer to one or more (i.e., at least one) of the grammatical objects of the article. For example, "a pharmaceutical composition" refers to one pharmaceutical composition or more than one pharmaceutical composition.

The term "about" or "approximately" means that a numerical value is within an acceptable error range for the particular value determined by those of ordinary skill in the art, and the numerical value depends in part on how the measurement or determination is carried out (i.e., the limits of the measurement system). For example, "about" or "approximately" in the art can mean a standard deviation within 1 or exceeding 1. Alternatively, "about" or "approximately" represents a range of ±15%, ±10%, ±5% or ±1%. Furthermore, particularly for a biological system or process, the term can mean at most an order of magnitude or at most 5 times the value. In the present disclosure, unless otherwise stated, "about XX" or "approximately XX" or "substantially comprising XX" refers to a numeral value within an acceptable error range for the particular value "XX" (including the numeral value "XX" itself, as well as those within an acceptable error range for the determination of the numeral value by those of ordinary skill in the art).

As described herein, any percentage range, ratio range, or integer range shall be understood as including the value of any integer within the listed range and including, when appropriate, fractions thereof (such as one tenth and one hundredth of the integer) unless otherwise indicated. Throughout the present disclosure, unless the context dictates otherwise, the words "comprise/include", "comprises/includes" and "comprising/including" will be understood as comprising/including the steps or elements or a group of steps or elements but not excluding any other steps or elements or groups of steps or elements. "Consisting of..." means comprising and being limited to what follows the phrase "consisting of". Thus, the phrase "consisting of..." means that the listed elements are required or necessary and that no other elements can be present. "Substantially consisting of..." means comprising any listed element that follows the phrase and being limited to other elements that do not interfere with or are favorable for the listed elements' activity or effects as detailed in the present disclosure. Thus, the phrase "substantially consisting of..." means that the listed elements are required or necessary but other elements are optional and can be present or absent depending on whether they affect the listed elements' activity or effects. The term "TSLP" refers to thymic stromal lymphopoietin. The term "TSLP" includes variants, isoforms, homologs, orthologs, and paralogs. For example, in some cases, an antibody specific for a human TSLP protein can cross-react with TSLP proteins from a species other than human (e.g., monkey). In other embodiments, an antibody specific for a human TSLP protein can be completely specific for the human TSLP protein and does not cross-react with other species or other types of proteins, or can cross-react with TSLP derived from some other species but not all others.

The term "human TSLP" refers to a TSLP protein having a human amino acid sequence, e.g., the amino acid sequence of human TSLP having Genbank Accession No. NP_149024.1. The terms "monkey or rhesus TSLP" and "mouse TSLP" refer to monkey and mouse TSLP sequences, respectively, e.g., having amino acid sequences with Genbank Accession Nos. NP_001100503.1 and NP_067342.1, respectively.

The "antibody" is meant to include a full-length antibody and any antigen-binding fragments (i.e., antigen-binding portion) or single chains thereof. The conventional full-length antibody is a glycoprotein comprising two heavy (H) chains and two light (L) chains linked by disulfide bonds. Each heavy chain is composed of a heavy chain variable region (V_{H}) and a heavy chain constant region composed of three domains, C_{H1}, C_{H2}, and C_{H3}. Each light chain is composed of a light chain variable region (V_{L}) and a light chain constant region composed of a domain C_{L}. The V_{H} and V_{L} regions may also be divided into hypervariable regions, known as complementarity determining regions (CDRs), which are separated by more conserved framework regions (FRs). Each V_{H} and V_{L} are composed of three CDRs and four FRs, arranged from the amino-terminus to the carboxyl-terminus in the following order: FR1, CDR1, FR2, CDR2, FR3, CDR3, FR4. The variable regions of the heavy chains and light chains comprise binding domains that interact with antigens. The constant regions of the antibody can mediate the binding of immunoglobulins to host tissues or factors, including various cells of the immune system (e.g., effector cells) and the first component (C1q) of the classical complement system.

The "antigen-binding portion" of an antibody (or simply "antibody portion") refers to one or more fragments of the antibody that retain the ability to specifically bind to an antigen (e.g., a TSLP protein). It has been demonstrated that the antigen-binding function of an antibody can be performed by fragments of a full-length antibody. Examples of binding fragments encompassed within the "antigen-binding portion" of an antibody include: (i) a Fab fragment, a monovalent fragment composed of V_{L}, V_{H}, C_{L} and C_{H1}; (ii) a F(ab')₂ fragment, a bivalent fragment comprising two Fab fragments linked by a disulfide bond at the hinge region; (iii) an Fd fragment composed of V_{H} and C_{H1}; (iv) an Fv fragment composed of V_{L} and V_{H} of a single arm of the antibody; (v) a dAb fragment composed of V_{H} (Ward et al., (1989) Nature 341:544-546); (vi) isolated complementarity determining regions (CDRs); and (vii) a nanobody, a heavy chain variable region comprising a single variable domain and two constant domains. Furthermore, although the two domains V_{L} and V_{H} of the Fv fragment are encoded by different genes, they can be joined via a synthetic linker by recombinant means into a single chain protein in which V_{L} and V_{H} pair to form a monovalent molecule (referred to as single-chain Fc (scFv); see, e.g., Bird et al., (1988) Science 242:423-426; and Huston et al., (1988) Proc. Natl. Acad. Sci. USA 85:5879-5883). These single-chain antibodies are also encompassed within the term "antigen-binding portion" of the antibody. These antibody fragments can be obtained using conventional techniques known to those skilled in the art, and the fragments can be subjected to functional screening using the same method as full-length antibodies.

The "isolated antibody" refers to an antibody that is substantially free of other antibodies with different antigenic specificities (e.g., an isolated antibody that specifically binds to a TSLP protein is substantially free of antibodies that specifically bind to antigens other than the TSLP protein). However, an isolated antibody that specifically binds to human TSLP protein may cross-bind to other antigens, e.g., TSLP proteins from other species. Furthermore, the isolated antibody is substantially free of other cellular materials and/or chemical substances.

The "mouse antibody" is meant to include an antibody in which the framework region and CDRs in the variable region are derived from mouse germline immunoglobulin sequences. Furthermore, if the antibody comprises a constant region, the constant region is also derived from mouse germline immunoglobulin sequences. The mouse antibody of the present disclosure may comprise amino acid residues not encoded by mouse germline immunoglobulin sequences (e.g., mutations introduced by *in-vitro* random or point mutations, or by *in-vivo* somatic mutations). However, the term "mouse antibody" as used herein does not include antibodies in which CDR sequences from other mammalian species are inserted into the mouse framework region sequences.

The "chimeric antibody" refers to an antibody obtained by combining genetic substances of non-human origin with genetic substances of human origin. Or more generally, a chimeric antibody refers to an antibody that combines genetic substances of one species with genetic substances of another species.

The "humanized antibody" refers to an antibody from a non-human species whose protein sequence has been modified to increase its similarity to a naturally occurring human antibody. The "isotype" refers to the class of antibodies encoded by the heavy chain constant region genes (e.g., IgM or IgG1).

The "antigen-recognizing antibody" and the "antibody specific to antigen/antibody having specificity to antigen" are used herein interchangeably with the term "antibody specifically binding to antigen".

An antibody that "specifically binds to human TSLP" refers to an antibody that binds to a human TSLP protein (or possibly also TSLP proteins from one or more non-human species) but does not substantially bind to a non-TSLP protein. Preferably, the antibody binds to a human TSLP protein with "high affinity", i.e., a K_{D} value of 5.0 × 10⁻⁸ M or less, preferably 1.0 × 10⁻⁸ M or less, more preferably 7.0 × 10⁻⁹ M or less.

The "not substantially bind to" a protein or cell refers to not binding to the protein or cell, or not binding to it with high affinity, i.e., binding to the protein or cell with a K_{D} value of 1.0 × 10⁻⁶ M or greater, preferably 1.0 × 10⁻⁵ M or greater, more preferably 1.0 × 10⁻⁴ M or greater, more preferably 1.0 × 10⁻³ M or greater, more preferably 1.0 × 10⁻² M or greater.

The "high affinity" for IgG antibodies refers to a K_{D} value for the antigen of 1.0 × 10⁻⁶ M or less, preferably 5.0 × 10⁻⁸ M or less, more preferably 1.0 × 10⁻⁸ M or less, more preferably 7.0 × 10⁻⁹ M or less, more preferably 1.0 × 10⁻⁹ M or less. However, for other antibody isotypes, "high affinity" binding may be different. For example, "high affinity" binding of IgM isotype refers to a K_{D} value of 10⁻⁶ M or less, preferably 10⁻⁷ M or less, more preferably 10⁻⁸ M or less.

The term "K_{assoc}" or "Kₐ" refers to the association rate of a particular antibody-antigen interaction, and the term "K_{dis}" or "K_{d}" refers to the dissociation rate of a particular antibody-antigen interaction. The term "K_{D}" refers to the dissociation constant, which is derived from the ratio of K_{d} to Kₐ (i.e., K_{d}/Kₐ) and is expressed in molar concentration (M). The K_{D} value of an antibody can be measured using methods well known in the art, and a preferred method for determining the K_{D} value of an antibody comprises using surface plasmon resonance, preferably using a biosensor system, such as the Biacore^{™} system.

The term "EC₅₀", also known as half-maximal effective concentration, refers to the concentration of an antibody that induces a response halfway between the baseline and the maximum after a particular exposure time.

The term "IC₅₀", also known as half-maximal inhibitory concentration, refers to the concentration of an antibody that inhibits a specific biological or biochemical function by 50% relative to the case where the antibody is absent.

The "subject" includes any human or non-human animal. The "non-human animal" includes all vertebrates, such as mammals and non-mammals, such as non-human primates, sheep, dogs, cats, cows, horses, chickens, amphibians, and reptiles; although mammals such as non-human primates, sheep, dogs, cats, cows, and horses are preferred.

The term "therapeutically effective amount" refers to an amount sufficient to prevent or ameliorate symptoms related to diseases or conditions (e.g., TSLP-related diseases), and/or reduce the severity of diseases or conditions. It should be understood that the therapeutically effective amount is related to the disease to be treated, wherein the actual effective amount can be readily determined by those skilled in the art.

### Anti-TSLP Antibodies in Pharmaceutical Composition of the Present Disclosure

The anti-TSLP antibody or the antigen-binding portion thereof in the pharmaceutical composition of the present disclosure may be an antibody having the structural and chemical characteristics as described below and in the examples. The amino acids SEQ ID NOs of the heavy/light chain variable regions of the antibodies are summarized in Table 1 below, some antibodies having the same VH or VL. The heavy chain constant region of the antibody may be a human IgG1 heavy chain constant region having an amino acid sequence set forth in SEQ ID NO: 12 or 37, or a human IgG4 heavy chain constant region having an amino acid sequence set forth in SEQ ID NO: 13, and the light chain constant region of the antibody may be a human κ light chain constant region having an amino acid sequence set forth in SEQ ID NO: 14. These antibodies may also comprise a mouse IgG1 or IgG2 heavy chain constant region and/or a mouse κ light chain constant region. The antibody may be composed of two heavy chains and two light chains linked by disulfide bonds, the C-terminus of the heavy chain variable region being linked to the N-terminus of the heavy chain constant region, and the C-terminus of the light chain variable region being linked to the N-terminus of the light chain constant region.

The heavy chain variable region CDRs and light chain variable region CDRs in Table 1 have been defined by the Kabat numbering system. However, as is well known in the art, the CDRs may also be determined by other numbering systems such as the Chothia, IMGT, AbM or Contact numbering system/method based on the heavy/light chain variable region sequence.

The VH and VL sequences (or CDR sequences) of other anti-TSLP antibodies that bind to human TSLP may be "mixed and paired" with the VH and VL sequences (or CDR sequences) of the anti-TSLP antibody in the pharmaceutical composition of the present disclosure. Preferably, when VH and VL chains (or CDRs in these chains) are mixed and paired, the VH sequence in a particular VH/VL pair is substituted with a structurally similar VH sequence. Likewise, it is preferred to replace the VL sequence in a particular VH/VL pair with a structurally similar VL sequence.

Therefore, in one embodiment, an anti-TSLP antibody or an antigen-binding portion thereof in the pharmaceutical composition of the present disclosure comprises:
(a) a heavy chain variable region comprising an amino acid sequence listed in Table 1; and
(b) a light chain variable region comprising an amino acid sequence listed in Table 1, or the VL of another anti-TSLP antibody, wherein the antibody specifically binds to human TSLP.

**Table 1. Amino acid SEQ ID NOs of heavy/light chain variable regions**

| Antibody | Heavy chain | | | | Light chain | | | |
|---|---|---|---|---|---|---|---|---|
| | V_{H} CDR1 | V_{H} CDR2 | V_{H} CDR3 | V_{H} | V_{L} CDR1 | V_{L} CDR2 | V_{L} CDR3 | V_{L} |
| Mouse/chimeric 1C5F12E9 | | | | SEQ ID NO.: 7 | | | | SEQ ID NO.: 10 |
| hu1C5F12E9-V1 | | | | SEQ ID NO.: 8 | | | | SEQ ID NO: 11, Xl-S, X2=V |
| hu1C5F12E9-V2 | | | | SEQ ID NO.: 9, Xl-R, X2-V, X3=R | | | | SEQ ID NO: 11, Xl-S, X2=V |
| hu1C5F12E9-V3 | | | | SEQ ID NO: 9, Xl=R, X2=V, X3=V | | | | SEQ ID NO: 11, Xl-S, X2=V |
| hu1C5F12E9-V4 | | | | SEQ ID NO: 9, Xl-R, X2=A, X3-R | | | | SEQ ID NO: 11, Xl-S, X2=V |
| hu1C5F12E9-V5 | | | | SEQ ID NO: 9, Xl=K, X2=A, X3=R | | | | SEQ ID NO: 11, Xl-S, X2=V |
| hu1C5F12E9-V6 | | | | SEQ ID NO: 9, Xl=K, X2=A, X3=V | | | | SEQ ID NO: 11, Xl-S, X2=V |
| hu1C5F12E9-V7 | | | | SEQ ID NO.: 8 | | | | SEQ ID NO: 11, Xl-A, X2=I |
| hu1C5F12E9-V8 | | | | SEQ ID NO.: 9, Xl-R, X2=V, X3=R | | | | SEQ ID NO: 11, Xl-A, X2=I |
| hu1C5F12E9-V9 | SEQ ID NO.: 1 | SEQ ID NO.: 2 | SEQ ID NO.: 3 | SEQ ID NO: 9, Xl=R, X2=V, X3=V | SEQ ID NO.: 4 | SEQ ID NO.: 5 | SEQ ID NO.: 6 | SEQ ID NO: 11, X1=A, X2=I |
| hu1C5F12E9-V10 | | | | SEQ ID NO: 9, Xl-R, X2=A, X3-R | | | | SEQ ID NO: 11, Xl-A, X2=I |
| hu1C5F12E9-V11 | | | | SEQ ID NO: 9, Xl=K, X2=A, X3=R | | | | SEQ ID NO: 11, X1=A, X2=I |
| hu1C5F12E9-V12 | | | | SEQ ID NO: 9, Xl=K, X2=A, X3=V | | | | SEQ ID NO: 11, Xl-A, X2=I |
| hu1C5F12E9-V13 | | | | SEQ ID NO.: 8 | | | | SEQ ID NO: 11, Xl=S, X2=I |
| hu1C5F12E9-V14 | | | | SEQ ID NO.: 9, Xl-R, X2=V, X3=R | | | | SEQ ID NO: 11, Xl=S, X2=I |
| hu1C5F12E9-V15 | | | | SEQ ID NO: 9, Xl=R, X2=V, X3=V | | | | SEQ ID NO: 11, Xl-S, X2=I |
| hu1C5F12E9-V16 | | | | SEQ ID NO: 9, Xl-R, X2=A, X3-R | | | | SEQ ID NO: 11, Xl=S, X2=I |
| hu1C5F12E9-V17 | | | | SEQ ID NO: 9, Xl=K, X2=A, X3=R | | | | SEQ ID NO: 11, Xl=S, X2=I |
| hu1C5F12E9-V18 | | | | SEQ ID NO: 9, Xl=K, X2=A, X3=V | | | | SEQ ID NO: 11, Xl-S, X2=I |

In another embodiment, an anti-TSLP antibody or an antigen-binding portion thereof in the pharmaceutical composition of the present disclosure comprises:
(a) CDR1, CDR2 and CDR3 of a heavy chain variable region listed in Table 1; and
(b) CDR1, CDR2 and CDR3 of a light chain variable region listed in Table 1, or the CDRs of another anti-TSLP antibody, wherein the antibody specifically binds to human TSLP.

In another embodiment, the anti-TSLP antibody or the antigen-binding portion thereof in the pharmaceutical composition of the present disclosure comprises the heavy chain variable region CDR2 of the anti-TSLP antibody, and the CDRs of other antibodies that bind to human TSLP, e.g., CDR1 and/or CDR3 of the heavy chain variable region, and/or CDR1, CDR2 and/or CDR3 of the light chain variable region of another anti-TSLP antibody.

It is well known in the art that, independent of the CDR1 and/or CDR2 domains, the CDR3 domains can individually determine the binding specificity of an antibody to a cognate antigen, and that multiple antibodies with the same binding specificity can be predictively generated based on a common CDR3 sequence.

In another embodiment, the anti-TSLP antibody in the pharmaceutical composition of the present disclosure comprises CDR2 of the heavy chain variable region of the anti-TSLP antibody and at least CDR3 of the heavy chain variable region and/or the light chain variable region of the anti-TSLP antibody, or CDR3 of the heavy chain variable region and/or the light chain variable region of another anti-TSLP antibody, wherein the antibody can specifically bind to human TSLP. These antibodies preferably (a) compete with the anti-TSLP antibody in the pharmaceutical composition of the present disclosure for binding to TSLP; (b) retain functional characteristics; (c) bind to the same epitope as the anti-TSLP antibody in the pharmaceutical composition of the present disclosure; and/or (d) have similar binding affinities as the anti-TSLP antibody in the pharmaceutical composition of the present disclosure. In another embodiment, the anti-TSLP antibody in the pharmaceutical composition of the present disclosure may further comprise the light chain variable region CDR2 of an anti-TSLP antibody, or the light chain variable region CDR2 of another anti-TSLP antibody, wherein the antibody specifically binds to human TSLP. In another embodiment, the anti-TSLP antibody in the pharmaceutical composition of the present disclosure may further comprise the heavy chain variable region and/or light chain variable region CDR1 of an anti-TSLP antibody, or the heavy chain variable region and/or light chain variable region CDR1 of another anti-TSLP antibody, wherein the antibody specifically binds to human TSLP.

### Conservative modification

In another embodiment, the anti-TSLP antibody in the pharmaceutical composition of the present disclosure comprises CDR1, CDR2, and CDR3 sequences that are different from the heavy chain variable region and/or the light chain variable region of the anti-TSLP antibody in the pharmaceutical composition of the present disclosure, the difference being derived from one or more conservative modifications. It should be understood in the art that some conservative sequence modifications do not eliminate the antigen-binding ability. See, e.g., Brummell et al., (1993) Biochem 32:1180-8; de Wildt et al., (1997) Prot. Eng. 10:835-41; Komissarov et al., (1997) J. Biol. Chem. 272:26864-26870; Hall et al., (1992) J. Immunol. 149:1605-12; Kelley and O'Connell (1993) Biochem.32:6862-35*;* Adib-Conquy et al., (1998) Int. Immunol.10:341-6 and Beers et al., (2000) Clin. Can. Res. 6:2835-43. Therefore, in one embodiment, the anti-TSLP antibody in the pharmaceutical composition of the present disclosure comprises a heavy chain variable region and/or a light chain variable region, the heavy chain variable region and the light chain variable region each comprising CDR1, CDR2 and CDR3, wherein:
(a) the CDR1 of the heavy chain variable region comprises the sequences listed in Table 1, and/or conservative modifications thereof; and/or
(b) the CDR2 of the heavy chain variable region comprises the sequences listed in Table 1, and/or conservative modifications thereof; and/or
(c) the CDR3 of the heavy chain variable region comprises the sequences listed in Table 1, and/or conservative modifications thereof; and/or
(d) the CDR1 and/or CDR2 and/or CDR3 of the light chain variable region comprise the sequences listed in Table 1, and/or conservative modifications thereof; and
(e) the antibody specifically binds to human TSLP.

The anti-TSLP antibody in the pharmaceutical composition of the present disclosure has one or more of the following functional characteristics, such as high affinity for human TSLP.

In various embodiments, the anti-TSLP antibody in the pharmaceutical composition of the present disclosure may be, for example, mouse, human, chimeric antibody, or humanized antibody.

The term "conservative sequence modification" as used herein refers to an amino acid modification that does not significantly affect or alter the binding characteristic of the antibody. Such conservative modifications include amino acid substitutions, additions and deletions. Modifications can be introduced into the anti-TSLP antibody in the pharmaceutical composition of the present disclosure by using standard techniques known in the art, such as point mutation and PCR-mediated mutation. Conservative amino acid substitutions are those in which an amino acid residue is replaced with an amino acid residue having a similar side chain. Amino acid residue groups having similar side chains are known in the art. Therefore, one or more amino acid residues in CDRs of the anti-TSLP antibody in the pharmaceutical composition of the present disclosure can be replaced with other amino acid residues from the same side chain group, and the resulting antibody can be functionally tested using the functional assays described herein. Methods for preparing and purifying an anti-TSLP antibody of the present disclosure are described in PCT/CN2020/113289, which is incorporated herein by reference in its entirety.

For clarity, the present disclosure is further described with the following examples, which are, however, not intended to limit the scope of the present disclosure. The anti-TSLP antibody 1C5F12E9 in the examples is antibody 1C5F12E9 of PCT/CN2020/113289, wherein mouse anti-TSLP antibody 1C5F12E9 was generated by hybridoma technology as described in PCT/CN2020/113289 and was obtained by *in-vitro* functional screening.

### Example 1: Humanization of Anti-TSLP Mouse Monoclonal Antibodies

Mouse anti-TSLP antibody 1C5F12E9 was humanized. Humanization of mouse antibodies was performed using an established CDR grafting method, as described below. To select a receptor skeleton for humanization of the mouse antibody 1C5F12E9, the sequences of the light and heavy chain variable regions of the antibody, together with the human immunoglobulin gene database, were subjected to basic local alignment search using BLAST. The human germline antibody having the highest homology with the mouse antibody was selected as the humanized receptor skeleton. The CDRs of the heavy/light chain variable region of the mouse antibody were inserted into the selected skeleton and further residues in the skeleton were mutated to obtain more candidate heavy/light chain variable regions. A total of 18 exemplary humanized 1C5F12E9 antibodies were obtained, namely hu1C5F12E9-V1 to hu1C5F12E9-V18, whose heavy/light chain variable region sequences are shown in Table 1.

Vectors comprising the heavy chain variable region encoding humanized 1C5F12E9 and the human IgG4 heavy chain constant region (SEQ ID NO: 13) as well as vectors comprising the light chain variable region of humanized 1C5F12E9 and the human κ light chain constant region (SEQ ID NO: 14) were constructed, and the vectors were transiently transfected into 50 mL of 293F suspension cells at a ratio of 60% light chain construct to 40% heavy chain construct using 1 mg/mL of PEI. After six days of culture in a shake flask, cell supernatants containing the humanized antibodies were collected, the cells in the supernatants were precipitated by centrifugation, and then the above-mentioned 18 antibodies were purified from the cell supernatants.

### Example 2: Characterization of Exemplary Humanized Anti-TSLP Monoclonal Antibodies

The binding affinity and binding kinetics of the purified exemplary humanized 1C5F12E9 antibody to human TSLP was assessed by using the Biacore T200 system (GE Healthcare, Pittsburgh, PA, USA).

Briefly, internally-synthesized recombinant human TSLP-his (SEQ ID NO: 28) or cynomolgus monkey TSLP-his protein (SEQ ID NO: 29) was dissolved in CH3COONa buffer (provided by Biocore) at a final concentration of 10 µg/mL and then covalently linked to a CM5 chip (carboxymethylated dextran coated chip, GE Healthcare # BR100530) via primary amine groups using a standard amine coupling kit provided by Biacore (GE Healthcare, Pittsburgh, PA, USA). Unreacted portions on the biosensor surface were blocked with ethanolamine. Then, a serially-diluted purified humanized 1C5F12E9 antibody (serially diluted 2-fold from an initial concentration of 100 nM in an HBS-EP+ buffer) and the benchmark Tezepelumab (also known as TSLP-BM, prepared in-house using the heavy and light chains set forth in SEQ ID NO: 35 and 36, serially diluted 2-fold from an initial concentration of 100 nM in an HBS-EP + buffer) were flowed over the chip at a flow rate of 50 µL/min, respectively. The antigen-antibody binding kinetics was followed for 4 min and the dissociation kinetics was followed for 13 min. Association and dissociation curves were fitted to a 1:1 Langmuir binding model using BIAcore evaluation software, and KD, Ka and Kd values were measured. The results are shown in Table 2 below.

**Table 2. Binding affinity of humanized 1C5F12E9 monoclonal antibody**

| mAb | Biacore kinetics | | |
|---|---|---|---|
| | Human TSLP | | |
| | Kₐ (M⁻¹s⁻¹) | K_{d} (s⁻¹) | K_{D} (M) |
| hu1C5F12E9-V1 | 9.93E+04 | 9.07E-08 (<1.00E-05) | <1.01E-10 |
| hu1C5F12E9-V3 | 1.03E+05 | 1.94E-08 (<1.00E-05) | <9.71E-11 |
| hu1C5F12E9-V5 | 1.03E+05 | 7.89E-08 (<1.00E-05) | <9.71E-11 |
| hu1C5F12E9-V7 | 1.08E+05 | 3.16E-08 (<1.00E-05) | <9.26E-11 |
| hu1C5F12E9-V9 | 1.22E+05 | 6.57E-07 (<1.00E-05) | <8.20E-11 |
| hu1C5F12E9-V11 | 9.07E+04 | 7.56E-07 (<1.00E-05) | <1.10E-10 |
| hu1C5F12E9-V12 | 9.61E+04 | 5.30E-08 (<1.00E-05) | <1.04E-10 |
| hu1C5F12E9-V13 | 1.04E+05 | 3.62E-07 (<1.00E-05) | <9.62E-11 |
| hu1C5F12E9-V14 | 1.05E+05 | 3.21E-06 (<1.00E-05) | <9.56E-11 |
| hu1C5F12E9-V15 | 1.12E+05 | 3.63E-06 (<1.00E-05) | <8.93E-11 |
| hu1C5F12E9-V16 | 1.04E+05 | 1.17E-06 (<1.00E-05) | <9.62E-11 |
| hu1C5F12E9-V17 | 1.10E+05 | 6.43E-06 (<1.00E-05) | <9.09E-11 |
| hu1C5F12E9-V18 | 1.21E+05 | 9.65E-06 (<1.00E-05) | <8.26E-11 |
| Chimeric 1C5F12E9 | 6.96E+04 | 6.36E-05 | 9.14E-10 |
| Tezepelumab | 1.99E+05 | 1.62E-04 | 8.15E-10 |

The Kd value lower limit for Biacore measurements is 1.00E-05, and Kd values below 1.00E-05 can be roughly calculated from the corresponding sensorgrams. The results indicate that all humanized 1C5F12E9 antibodies have a higher binding affinity for human TSLP than Tezepelumab.

### Example 3: Characterization of Humanized Anti-TSLP Antibodies hu1C5F12E9-V8 and hu1C5F12E9-V14

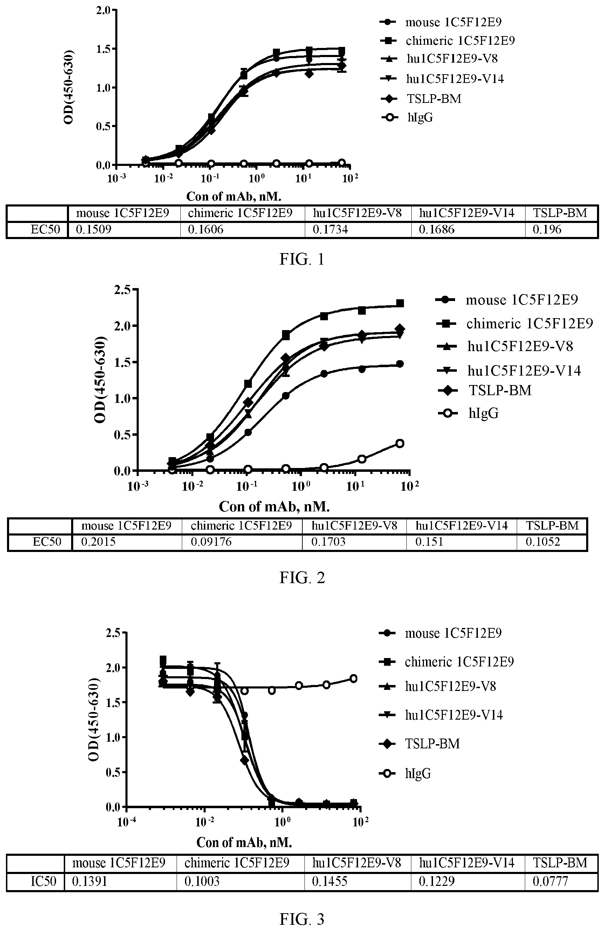

Humanized antibodies hu1C5F12E9-V8 and hu1C5F12E9-V14 were selected for further characterization. Specifically, the binding affinity/ability of the antibodies for human and cynomolgus monkey TSLP as well as other functions thereof were determined by using Biacore, capture ELISA, indirect ELISA, competitive ELISA, cell-based ligand blocking FACS and cell-based functional assays as in Example 2 and method below, and the results are shown in Table 3 below, and FIGs. 1-5 and 6A-6B.

For capture ELISA, 2 µg/mL of affinity purified goat anti-human IgG antibody dissolved in PBS (Jackson Immuno Research, 109-005-098) was coated in a 96-well plate at 100 µL/well and incubated overnight at 4 °C. The plate was washed 4 times with a washing buffer (PBS + 0.05% Tween-20, PBST) and then blocked at 37 °C for 2 h by adding 200 µL of blocking buffer (PBST containing 5% w/v skim milk) per well. The plate was washed again, added with the humanized anti-TSLP antibody, Tezepelumab or hIgG of the present disclosure (Hualan Biological Engineering Inc.) serially diluted at 100 µL/well (from an initial concentration of 66.7 nM, serially diluted 5-fold in PBST containing 2.5% skim milk), incubated for 40 min at 37 °C, and then washed again for 4 times. The 96-well plate with capture antibodies was added with biotin-labeled human TSLP-his protein (SEQ ID NO: 28, prepared in-house, dissolved in PBST containing 2.5% skim milk, at a final concentration of 0.23 nM) at 100 µL/well, incubated at 37 °C for 40 min, washed for 4 times, and then added with 100 µL of HRP-labeled streptavidin (diluted at 1:10000 in PBST, Jackson Immuno Research, 016-030-084) per well, and incubated at 37 °C for 40 min. After the last wash, the plate was added with TMB (Innoreagents) at 100 µL/well for incubation. After 15 min, the reaction was stopped by adding 1 M H₂SO₄ at 50 µL/well at room temperature, and the absorbance of each well was read on a microplate reader using the dual wavelength mode (TMB detection wavelength: 450 nm, reference wavelength: 630 nm). Plotting was performed using OD (450-630) values and the corresponding antibody concentrations. Data were analyzed using Graphpad Prism software, and EC50 values were obtained. The results for some antibodies are shown in FIG. 1.

For indirect ELISA, a 96-well plate was coated with 2 µg/mL of cynomolgus monkey TSLP-his protein (SEQ ID NO: 29, prepared in-house) dissolved in carbonate/bicarbonate buffer (pH 9.6) at 100 µL/well and incubated overnight at 4 °C. The plate was washed 4 times with a washing buffer (PBS + 0.05% Tween-20, PBST) and then blocked at 37 °C for 2 h by adding 200 µL of blocking buffer (PBST containing 5% w/v skim milk) per well. The plate was washed again, and added with the humanized anti-TSLP antibody, Tezepelumab or hIgG of the present disclosure serially diluted at 100 µL/well (serially diluted 5-fold from an initial concentration of 66.7 nM in PBST containing 2.5% skim milk), and then incubated at 37 °C for 40 min. The plate was washed again for 4 times, and added with peroxidase-labeled affinity purified F(ab')₂ fragmented goat anti-human IgG antibody (Jackson Immunoresearch, 109-036-098) at 100 µL/well, and then incubated at 37 °C for 40 min. After the last wash, the plate was added with TMB (Innoreagents) at 100 µL/well for incubation. After 15 min, the reaction was stopped by adding 1 M H₂SO₄ at 50 µL/well at room temperature, and the absorbance of each well was read on a microplate reader using the dual wavelength mode (TMB detection wavelength: 450 nm, reference wavelength: 630 nm). Plotting was performed using OD (450-630) values and the corresponding antibody concentrations. Data were analyzed using Graphpad Prism software, and EC50 values were obtained. The results for some antibodies are shown in FIG. 2.

The ability of the humanized anti-TSLP antibodies to block the binding of TSLP to TSLPR/IL7R was determined using competitive ELISA. Briefly, TSLPR-Fc protein (SEQ ID NO: 30, prepared in-house) was dissolved in PBS at a final concentration of 1 µg/mL, IL7Ra-Fc protein (SEQ ID NO: 31, prepared in-house) was dissolved in PBS at a final concentration of 1 µg/mL, and a 96-well plate was coated with the two solutions (100 µL each) and incubated overnight at 4 °C. The next day, the plate was washed with a washing buffer (PBS + 0.05% Tween-20, PBST), added with PBST containing 5% w/v of skim milk, and blocked at 37 °C for 2 h. The plate was then washed with a washing buffer. The humanized anti-TSLP antibody or control was diluted with biotin-labeled human TSLP-Fc (SEQ ID NO: 32, prepared in-house, dissolved in PBST containing 2.5% skim milk, at a final concentration of 0.29 nM), serially diluted 3-fold from an initial concentration of 66.7 nM, and incubated at room temperature for 40 min. Then, the antibody/TSLP-Fc mixture was added to the plate coated with TSLPR/IL7R at 100 µL/well. After incubation at 37 °C for 40 min, the plate was washed for 4 times with a washing buffer. HRP-labeled streptavidin was then added, and the plate was incubated at 37 °C for 40 min to detect biotin-labeled human TSLP-Fc bound to TSLPR/IL7R. The plate was washed again with a washing buffer. Finally, TMB was added, and the reaction was stopped with 1 M H₂SO₄. The absorbance of each well was read on a microplate reader using the dual wavelength mode (TMB detection wavelength: 450 nm, reference wavelength: 630 nm). Then, plotting was performed using OD (450-630) values and the corresponding antibody concentrations. Data were analyzed using Graphpad Prism software, and IC₅₀ values were obtained. The results for some antibodies are shown in FIG. 3.

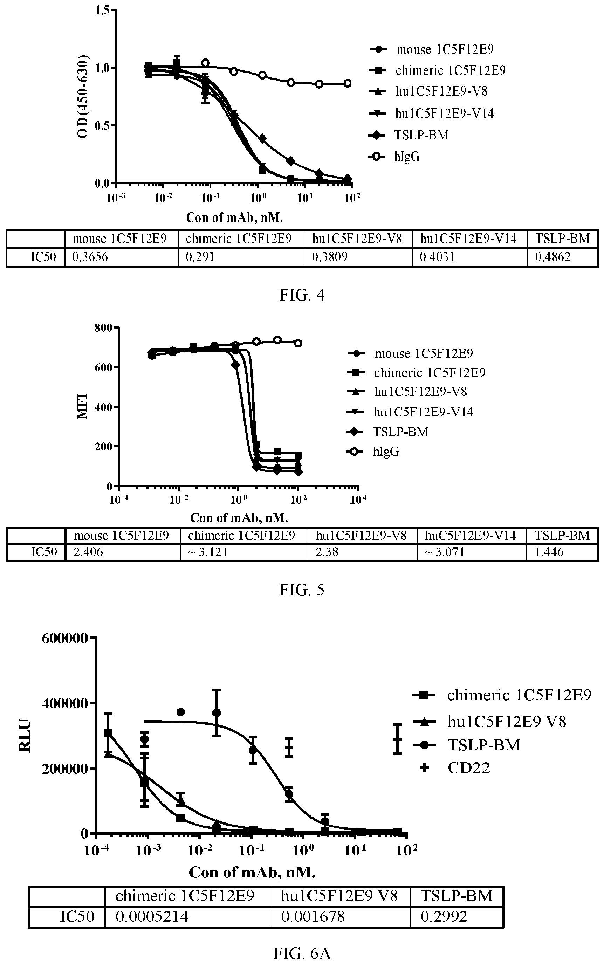

The ability of the humanized anti-TSLP antibody to block the binding of the benchmark (Tezepelumab) to human TSLP was determined using competitive ELISA. Briefly, a 96-well plate was coated with 2 µg/mL of Tezepelumab in PBS at 100 µL/well, and incubated overnight at 4 °C. The next day, the plate was washed with a washing buffer (PBS + 0.05% Tween-20, PBST), added with PBST containing 5% w/v of skim milk, and blocked at 37 °C for 2 h. Meanwhile, the anti-TSLP antibody or control was diluted with biotin-labeled human TSLP-Fc (SEQ ID NO: 32, dissolved in PBST containing 2.5% skim milk, at a final concentration of 0.047 nM), serially diluted 4-fold from an initial concentration of 40 nM, and incubated at room temperature for 40 min. Then, the antibody/TSLP-Fc-biotin mixture was added to the 96-well plate coated with the benchmark at 100 µL/well. After incubation at 37 °C for 40 min, the plate was washed for 4 times with a washing buffer. HRP-labeled streptavidin was then added, and the plate was incubated at 37 °C for 40 min to detect biotin-labeled human TSLP-Fc bound to the benchmark. Finally, the plate was washed with a washing buffer. TMB was added, and the reaction was stopped with 1 M H₂SO₄. The absorbance of each well was read on a microplate reader using the dual wavelength mode (TMB detection wavelength: 450 nm, reference wavelength: 630 nm). Then, plotting was performed using OD (450-630) values and the corresponding antibody concentrations. Data were analyzed using Graphpad Prism software, and IC₅₀ values were obtained. The results for some antibodies are shown in FIG. 4.

In cell-based ligand blocking FACS, using flow cytometry (FACS), the activity of the humanized anti-TSLP antibody to block the binding of TSLP-Fc protein to the cell surface TSLPR/IL7R was assessed using the cell line BAF3-3E6, whose cell surface expresses human TSLPR (amino acid residues 1-371 of uniprot No. Q9HC73.1, SEQ ID NO: 33) and human IL7R (amino acid residues 1-459 of uniprot No. P16871.1, SEQ ID NO: 34). According to the instructions of the lipofectamine 3000 transfection reagent (Thermo Fisher), BAF3 cells (iCell Bioscience Inc., MIMCL-021) were transfected with recombinant plasmid pCMV-T-P (inserting the TSLPR coding sequence between EcoRI and Xbal sites) and recombinant plasmid pCMV3-SP (inserting the IL7R coding sequence between HindIII and Xbal) to prepare a BAF3-3E6 cell line. Briefly, the anti-TSLP antibody of the present disclosure, the benchmark, or the negative control hIgG (human immunoglobulin for intravenous injection (pH 4) (Hualan Biological Engineering Inc.)) was diluted with a human TSLP-Fc solution (SEQ ID NO: 32, prepared in-house, dissolved in a FACS buffer, at a final concentration of 0.38 nM), serially diluted 2-fold from an initial concentration of 30 nM, and incubated at room temperature for 40 min. BAF3-3E6 cells were harvested from cell culture flasks, washed twice, and then resuspended in phosphate buffered saline (PBS) containing 2% v/v of fetal bovine serum (FACS buffer). A 96-well plate containing 1 × 10⁵ cells/well was added with an antibody/TSLP-Fc-biotin mixture at 100 µL/well, and incubated at 4 °C for 40 min. The cells were washed twice with a FACS buffer, then added with R-phycoerythrin-labeled streptavidin (diluted at 1:1000 in a FACS buffer, Jackson Immunoresearch, 016-110-084) at 100 µL/well, and incubated at 4 °C for 40 min in the dark. The cells were washed twice and resuspended in a FACS buffer. Fluorescence values were measured using Becton Dickinson FACS Canto II-HTS. Data were analyzed using Graphpad Prism software, and IC₅₀ values were obtained. The results for some antibodies are shown in FIG. 5.

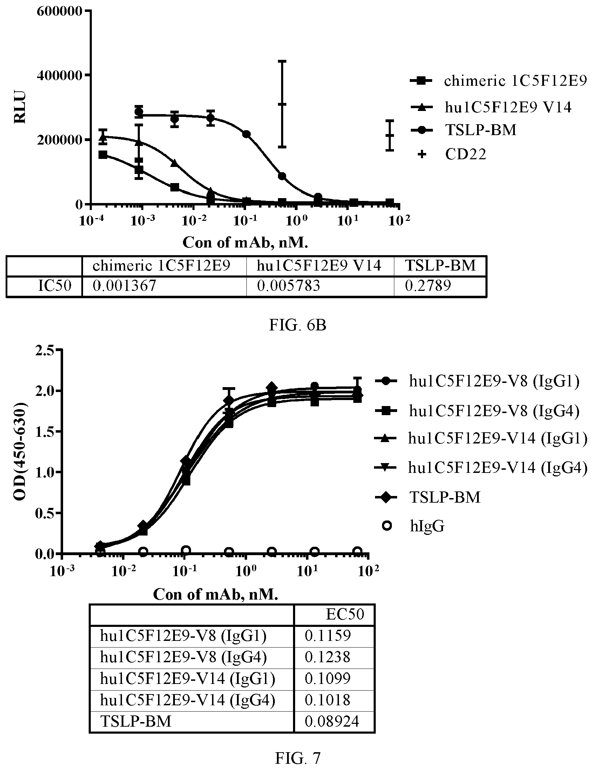

The proliferation and survival of BAF3 cells were generally dependent on IL-3. However, when these cells were engineered to express both human TSLPR and human IL7R, and TSLP was added to the cell culture media, they could survive in the absence of IL-3. Using a method for cell-based functional assays, the inhibitory activity of the humanized anti-TSLP antibodies against the proliferation of BAF3-3E6 cells expressing TSLPR (SEQ ID NO: 33)/IL7R (SEQ ID NO: 34) was further tested. Briefly, 100 µL of RPMI1640 media (Gibco, A10491-01) containing 8 × 10³ BAF3-3E6 cells in logarithmic growth phase were seeded onto a 96-well plate, wherein the RPMI1640 media contain 10% FBS (Gibco, A10099-141). Then, 50 µL of human TSLP-his protein (SEQ ID NO: 28, prepared in-house, dissolved in RPMI-1640, at a final concentration of 6.4 ng/mL) was mixed with 50 µL of humanized anti-TSLP antibody or control (serially diluted 5-fold from an initial concentration of 40 µg/mL), and the mixture was incubated at room temperature for 30 min. Then, a 96-well plate containing BAF3-3E6 cells was added with the antibody/TSLP-his mixture at 100 µL/well, and cultured at 37 °C for 72 h in an incubator containing CO₂. Thereafter, the 96-well plate containing cells was incubated with Cell Titer-Glo^{®} luminescent cell viability assay kit (Promega, G7572, 50 µL/well) at 37 °C for 10 min. The chemiluminescence values were measured using Tecan Infinite^{®} 200 Pro. Data were analyzed using Graphpad Prism software, and IC₅₀ values were obtained. The results for some antibodies are shown in FIGs. 6A-6B.

The data indicate that hu1C5F12E9-V8 and hu1C5F12E9-V14 showed comparable *in-vitro* activity to the parental mouse and chimeric antibodies.

As shown in Table 3, FIG. 1 and FIG. 2, the binding affinity/activity of the humanized antibodies hu1C5F12E9-V8 and hu1C5F12E9-V14 to human TSLP was higher than those of the benchmark, and the binding affinity/activity of the humanized antibodies hu1C5F12E9-V8 and hu1C5F12E9-V14 to cynomolgus monkey TSLP was comparable to those of the benchmark.

FIGs. 3 and 5 show that the humanized antibodies hu1C5F12E9-V8 and hu1C5F12E9-V14 were able to block the binding of human TSLP to human TSLPR/IL7R.

FIGs. 6A-6B show that the humanized antibodies hu1C5F12E9-V8 and hu1C5F12E9-V14 were able to block the interaction between TSLP and TSLPR/IL7R, resulting in the blockade of the TSLP pathway and death of BAF3-3E6 cells at low antibody concentrations, whereas the benchmark required high antibody levels to exert such potency.

**Table 3. Binding affinity of monoclonal antibodies hu1C5F12E9-V8 and hu1C5F12E9-V14**

| mAbs | BIAcore kinetics | | | | | |
|---|---|---|---|---|---|---|
| | Human TSLP | | | Cynomolgus monkey TSLP | | |
| | Kₐ | K_{d} | K_{D} | Kₐ | K_{d} | K_{D} |
| | (1/Ms) | (s-1) | (M) | (1/Ms) | (s-1) | (M) |
| Mouse 1C5F12E9 | 1.04E+05 | <1.00E-05 | <9.66E-11 | 2.89E+05 | <1.00E-05 | <3.46E-11 |
| Chimeric 1C5F12E9 | 1.93E+05 | <1.00E-05 | <5.17E-11 | 3.83E+05 | <1.00E-05 | <2.61E-11 |
| hu1C5F12E9-V8 | 1.67E+05 | <1.00E-05 | <5.98E-11 | 3.05E+05 | <1.00E-05 | <3.28E-11 |
| hu1C5F12E9-V14 | 1.64E+05 | <1.00E-05 | <6.10E-11 | 3.11E+05 | <1.00E-05 | <3.22E-11 |
| Tezepelumab | 4.24E+05 | 9.84E-05 | 2.32E-10 | 8.49E+05 | 5.71E-05 | 6.72E-11 |

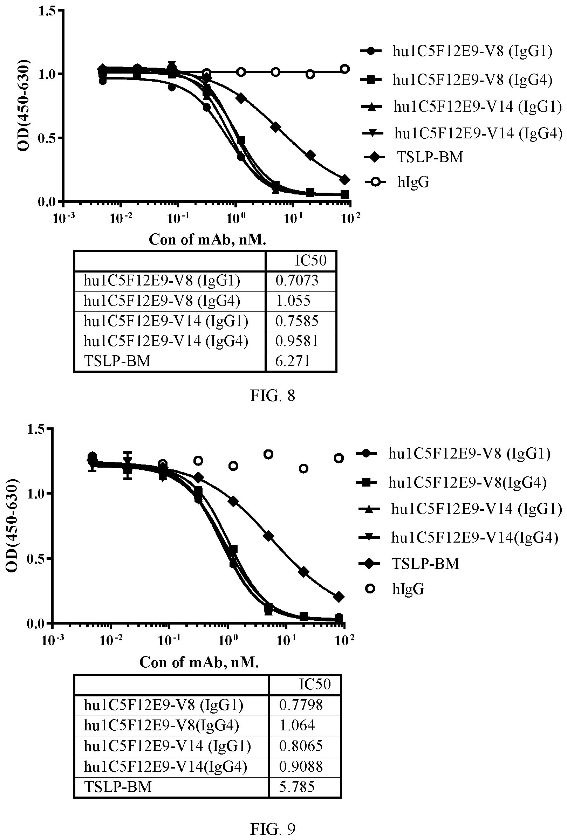

Thereafter, the binding affinity/ability of the humanized antibodies hu1C5F12E9-V8 and hu1C5F12E9-V14 having the human IgG1 heavy chain constant region (SEQ ID NO: 12) and the human κ light chain constant region (SEQ ID NO: 14) for human and cynomolgus monkey TSLP and the binding affinity/ability of hu1C5F12E9-V8 and hu 1 CS F 12E9-V 14 having the human IgG4 heavy chain constant region (SEQ ID NO: 13) and the human κ light chain constant region (SEQ ID NO: 14) for human and cynomolgus monkey TSLP, as well as other functions thereof were compared by using Biacore, capture ELISA, competitive ELISA, cell-based ligand blocking FACS, cell-based functional assays, cell-based reporter gene assays, and protein thermal shift assays as in Examples 2-3 and method below. The results are shown in FIGs. 7-10, 11A-11B, 12A-12B and 13.

To determine the thermal stability of the four anti-TSLP humanized antibodies, Tm (melting temperature) was measured in protein thermal shift assay using the GloMelt^{™} thermal shift protein stability kit (Biotium, 33022-T). Briefly, the GloMelt^{™} dye was thawed to room temperature. The vial containing the dye was vortexed and centrifuged. Then, 5 µL of 200× dye was added to 95 µL of PBS to prepare 10× dye. 2 µL of 10× dye and 10 µg of humanized antibodies were added to the reaction system, and PBS was added to a total reaction volume of 20 µL. The centrifuge tubes containing the dye and the antibodies were centrifuged briefly and placed in a real-time PCR thermal cycler (Roche, LightCycler 480 II) in which the parameters of the Melt Curve program are shown in Table 4.

**Table 4. Parameters of Melt Curve program**

| Step | Temperature | Heating rate | Duration of time |
|---|---|---|---|
| Initial hold | 25°C | NA | 30 s |
| Melt curve | 25-99°C | 0.1°C/s | NA |

In a cell-based reporter gene assay, the reporter gene cell line HEK293T-TSLPR/IL7R/STE1T5-Luc, whose cell surface expresses human TSLPR (SEQ ID NO: 33) and human IL7R (SEQ ID NO: 34), was used. According to the instructions of the lipofectamine 3000 transfection reagent (Thermo Fisher), HEK293T cells (ATCC^{®} CRL-11268) were transfected with recombinant plasmid pCMV-T-P (inserting the TSLPR coding sequence between EcoRI and Xbal sites), recombinant plasmid pCMV3-SP (inserting the IL7R coding sequence between HindIII and Xbal) and pGL4.52 [luc2P/STAT5RE/Hygro] (Promega) to prepare in-house HEK293T-TSLPR/IL7R/STAT5-Luc cells. Briefly, HEK293T-TSLPR/IL7R/STAT5-Luc cells were harvested from cell culture flasks. Then, 100 µL of DMEM media (Gibco, 10566-016) containing 5 × 10⁴ cells were seeded onto a 96-well cell culture plate (Corning, 30218026), wherein the DMEM media contain 10% FBS (Gibco, 10099-141). Meanwhile, 50 µL of human TSLP-his (SEQ ID NO: 28, dissolved in a DMEM medium containing 10% FBS, at a final concentration of 160 ng/mL) was mixed with 50 µL of serially-diluted anti-TSLP antibodies hu1C5F12E9-V8 (IgG1), hu1C5F12E9-V8 (IgG4), hu1C5F12E9-V14 (IgG1), hu1C5F12E9-V14 (IgG4) and Tezepelumab (serially-diluted 5-fold from an initial concentration of 200 µg/mL in a DMEM medium containing 10% FBS), respectively, and the mixture was incubated at room temperature for 30 min. Then, a 96-well cell culture plate was added with the anti-TSLP antibody/TSLP-his mixture at 100 µL/well, and incubated at 37 °C for 16-18 h in an incubator containing CO₂. 100 µL of supernatant was discarded per well, and then luciferase assay reagent (Promega, E6120) was added at 50µL/well. After 10 min, the plate was analyzed using a Tecan Infinite 200Pro plate reader. The data of luminescence signals were analyzed using Graphpad prism software, and IC₅₀ values were obtained.

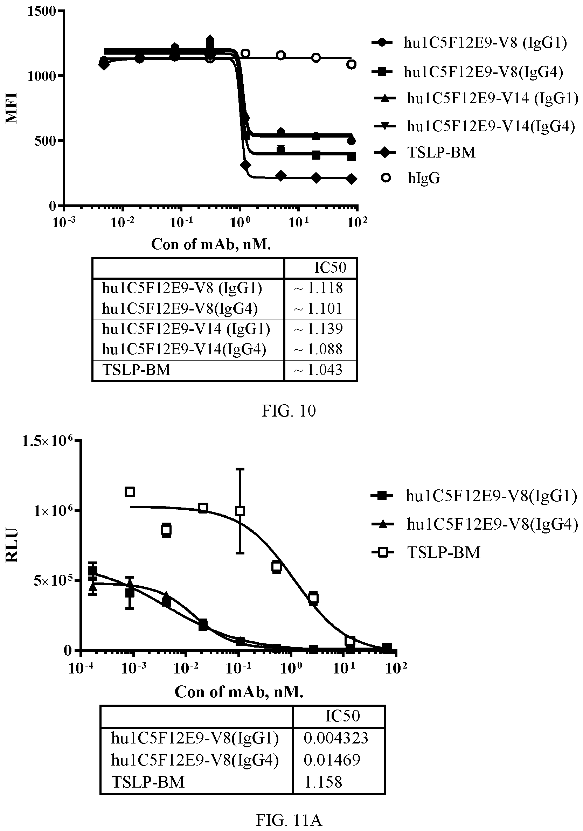

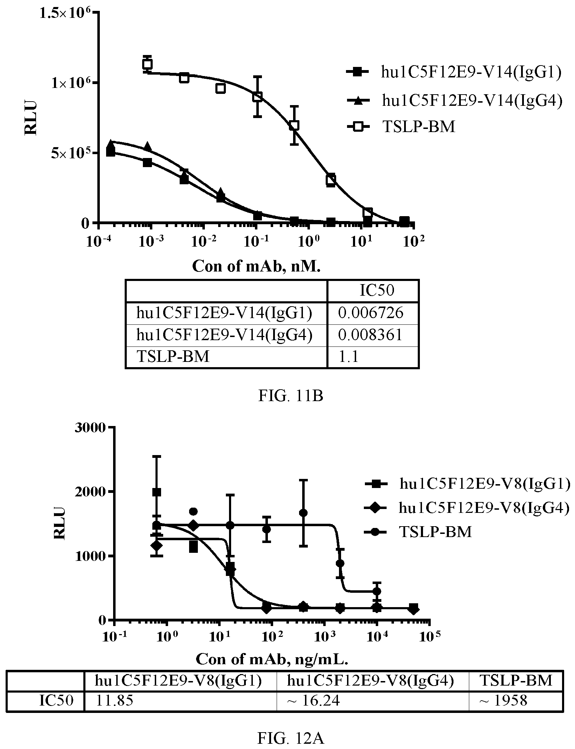

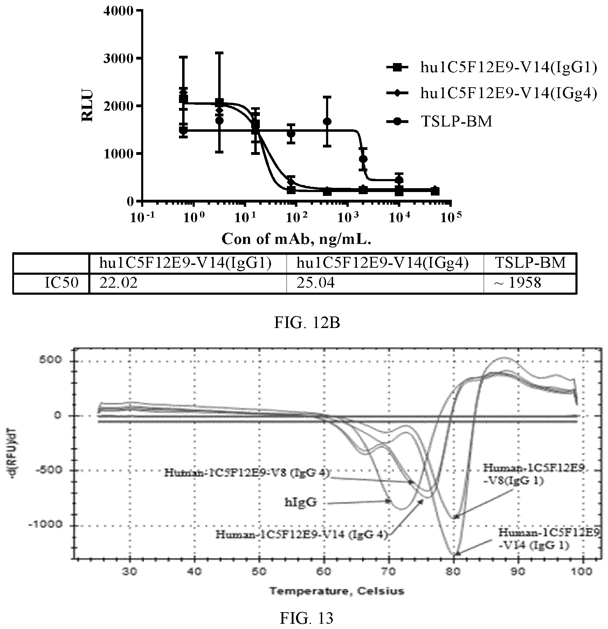

As shown in FIGs. 7, 8, 10, 11A-11B and 12A-12B, hu1C5F12E9-V8 (IgG1), hu1C5F12E9-V8 (IgG4), hu1C5F12E9-V14 (IgG1) and hu1C5F12E9-V14 (IgG4) had comparable or better *in-vitro* activity compared to Tezepelumab. In particular, as shown in FIGs. 11A-11B and 12A-12B, the humanized antibodies were able to block the interaction between TSLP and TSLPR/IL7R at antibody concentrations much lower than the benchmark concentration, resulting in the blockade of the TSLP pathway and death of BAF3-3E6 cells.

As shown in FIG. 13, the melting temperatures (T1, T2) of hu1C5F12E9-V8 (IgG1), hu1C5F12E9-V8 (IgG4), hu1C5F12E9-V14 (IgG1) and hu1C5F12E9-V14 (IgG4) were (69.5 °C, 80 °C), (66.5 °C, 76 °C), (69.5 °C, 80 °C) and (66.5 °C, 76 °C), respectively.

### Example 4: Screening of Pharmaceutical Compositions

Size exclusion chromatography (SEC-UPLC): for determining the purity of the antibodies in the sample; Thermo Vanquish F high performance liquid chromatography was used, Waters ACQUITY UPLC Protein BEH SEC Column (200Å) gel chromatography column were used, with Waters ACQUITY UPLC Protein BEH SEC Guard Column (200Å) as pre-column, elution was performed with 50 mmol/L of phosphate buffered saline-200 mmol/L of sodium chloride solution (pH 7.0) as mobile phase, and the detection wavelength was 280 nm. The contents of the high-molecular impurities and the immunoglobulin monomers were calculated in percentage using an area normalization method.

Differential scanning calorimetry (DSC): the sample was analyzed for the unfolding temperature (Tm) using MicroCal VP-Capillary DSC (Malvern) and diluted to a concentration of 1 mg/mL. The procedure was as follows: the starting temperature of scanning was 20 °C, the ending temperature of scanning was 110 °C, and the heating rate was 60 °C/h.

The aggregation temperature (Tagg) of the sample was measured using Dynaproplate Reader III (Wyatt), the sample was added to a 384-well sample plate, and the plate was sealed with a sealing film. The 384-well sample plate was centrifuged to remove air bubbles, and then the measurement was performed. The procedure was as follows: the temperature program was from 35 °C to 85 °C, and the scanning time was 5 seconds.

The samples were analyzed for viscosity using an HVROC-S viscometer (Rheosense), the Auto mode was selected, and the analysis results were recorded.

Capillary isoelectric focusing electrophoresis (CIEF): for determining the charge variants of the sample; a ProteinSimple iCE3 imaging capillary isoelectric focusing electrophoresis apparatus, a Maurice rapid full-automatic protein characterization and analysis system, a ProteinSimple coated silica capillary and an ultraviolet detector were adopted for detection at an ultraviolet detection wavelength of 280 nm. The sample injection time was set to 60 seconds, the pre-focusing time was set to 1 minute at 1500 V, and the focusing time was set to 5 minutes at 3000 V. The peak area of the charge variants was calculated in percentage using a peak area normalization method. CE-SDS reduction electrophoresis: a Beckman Coulter Pa 800 Plus biopharmaceutical analysis system, a non-coated fused silica capillary with an inner diameter of 50 µm, a total length of 31 cm and an effective length of 21 cm, and a PDA detector were adopted for detection at a detection wavelength of 220 nm. The corrected peak areas of light chain, heavy chain and non-glycosylated heavy chain were calculated in percentage using an area normalization method. CE-SDS non-reduction electrophoresis: a Beckman Coulter Pa 800 Plus biopharmaceutical analysis system, a non-coated fused silica capillary with an inner diameter of 50 µm, a total length of 31 cm and an effective length of 21 cm, and a PDA detector were adopted for detection at a detection wavelength of 220 nm. The corrected peak area of the main peak was calculated in percentage using an area normalization method.

The biological activity was detected using an enzyme-linked immunosorbent assay (ELISA), and a 96-well plate was coated with 2 µg/mL of TSLP protein (Sinobiological, 16135-H08H) at 100 µL/well, and incubated overnight at 2-8 °C. After washing, the 96-well plate was added with 250 µL of blocking solution (3% BSA in PBS) per well and incubated at 25 °C for 2 h. After washing, the 96-well plate was added with a benchmark and a test sample (serially diluted 4-fold from an initial concentration of 4000 ng/mL to obtain a total of 7 concentrations), respectively, and incubated at 25 °C for 2 h, wherein the benchmark was a pharmaceutical composition sample having process representativeness, and the test sample was a pharmaceutical composition sample to be tested. The preparation method is described below. After washing, the 96-well plate was added with an HRP-labeled goat anti-human antibody (PekinElmer, NEF802001EA, diluted according to the instruction) at 100 µL/well, and incubated at 25 °C for 1 h. After washing, the 96-well plate was added with 100 µL of TMB per well, and incubated at 25 °C for 5 min in the dark. Finally, 1 M H₂SO₄ was added to stop color development, and the plate was placed at room temperature for 5 min, followed by detection using a microplate reader. The absorbance was measured at a wavelength of 450 nm by taking 650 nm as a reference wavelength, and the measurement result was recorded. Biological activity of test sample (%) = (EC₅₀ value of benchmark/EC₅₀ value of test sample) × 100%

Preparation of a buffering agent: the sodium phosphate buffering agent was prepared from sodium dihydrogen phosphate monohydrate (NaH₂PO₄•H₂O) and disodium hydrogen phosphate dodecahydrate (Na₂HPO₄•12H₂O), for example, 20 mM of sodium phosphate buffering agent (pH 6.0) was prepared from about 2.54 g/L NaH₂PO₄•H₂O and about 0.573 g/L Na₂HPO₄•12H₂O. The sodium acetate buffering agent was prepared from anhydrous sodium acetate and acetic acid, for example, 20 mM of a sodium acetate buffering agent (pH 6.0) was prepared from about 1.58 g/L of anhydrous sodium acetate and about 0.116 g/L of acetic acid. The histidine buffering agent was prepared from L-histidine and adjusted to a target pH with acetic acid, for example, 20 mM of a histidine buffering agent (pH 6.0) was prepared from about 3.1 g/L of histidine and adjusted to a pH of 6 with acetic acid.

The anti-TSLP antibody (hu1C5F12E9-V8 (IgG1)', having the human IgG1 heavy chain constant region set forth in SEQ ID NO: 37 and the human κ light chain constant region set forth in SEQ ID NO: 14) was displaced into the screened buffering agent in Table 5 by ultrafiltration centrifugation, and after the displacement was completed, concentration was performed, and excipients were added according to the screened pharmaceutical compositions in Table 5, respectively. After mixing well, filtering and sterilizing were performed by passing through a 0.22 µm filter membrane. Tm values, Tagg values and SEC-UPLC analysis results for the different pharmaceutical compositions are shown in Table 5.

**Table 5. Tm, Tagg, SEC-UPLC analysis results of the screened pharmaceutical compositions**

| Pharmaceutical composition | Tm (°C) | Tagg (°C) | SEC-UPLC(%) | |
|---|---|---|---|---|
| | | | High-mol ecular impurities | Immunoglobulin monomers |
| 120 mg/mL of anti-TSLP antibody, 20 mM of sodium phosphate buffering agent, 200 mM of mannitol (36.43 g/L), 0.04% w/v of polysorbate 80, pH 5.0 | 70.08 | 62.19 | 0.49 | 99.2 |
| 120 mg/mL of anti-TSLP antibody, 20 mM of sodium phosphate buffering agent, 200 mM of mannitol (36.43 g/L), 0.04% w/v of polysorbate 80, pH 6.0 | 71.60 | 62.37 | 0.55 | 99.2 |
| 120 mg/mL of anti-TSLP antibody, 20 mM of sodium phosphate buffering agent, 200 mM of mannitol (36.43 g/L), 0.04% w/v of polysorbate 80, pH 7.0 | 72.08 | 61.57 | 0.47 | 99.3 |
| 120 mg/mL of anti-TSLP antibody, 20 mM of histidine buffering agent, 200 mM of mannitol (36.43 g/L), 0.04% w/v of polysorbate 80, pH 6.0 | 70.10 | 67.40 | 0.49 | 99.2 |
| 120 mg/mL of anti-TSLP antibody, 20 mM of sodium acetate buffering agent, 200 mM of mannitol (36.43 g/L), 0.04% w/v of polysorbate 80, pH 6.0 | 71.63 | 63.23 | 0.53 | 99.2 |

Illustratively, the histidine buffering agent was selected as the buffering agent for further screening, and the anti-TSLP antibody (hu1C5F12E9-V8 (IgG1)', having the human IgG1 heavy chain constant region set forth in SEQ ID NO: 37 and the human κ light chain constant region set forth in SEQ ID NO: 14) was displaced into the screened histidine buffering agent in Table 6 by ultrafiltration centrifugation, and after the displacement was completed, concentration was performed, and excipients were added according to the screened pharmaceutical compositions in Table 6, respectively. After mixing well, filtering and sterilizing were performed by passing through a 0.22 µm filter membrane. Tm values, Tagg values, viscosities, and SEC-UPLC analysis results for the different pharmaceutical compositions are shown in Table 6.

**Table 6. Tm, Tagg, viscosity and SEC-UPLC analysis results of the screened pharmaceutical compositions**

| Pharmaceutical composition | Tm (°C) | Tagg (°C) | SEC-UPLC(%) | | Viscosity (mpa·s) |
|---|---|---|---|---|---|
| | | | High-mol ecular impurities | Immunoglobulin monomers | |
| 120 mg/mL of anti-TSLP antibody, 20 mM of histidine buffering agent, 200 mM of mannitol, 0.02% w/v of polysorbate 80, pH 5.5 | 68.97 | 64.78 | 0.42 | 98.6 | 8.365 |
| 120 mg/mL of anti-TSLP antibody, 20 mM of histidine buffering agent, 200 mM of mannitol, 0.02% w/v of polysorbate 80, pH 6.0 | 70.22 | 66.40 | 0.47 | 98.5 | 9.679 |
| 120 mg/mL of anti-TSLP antibody, 20 mM of histidine buffering agent, 200 mM of mannitol, 0.02% w/v of polysorbate 80, pH 6.5 | 71.99 | 64.75 | 0.39 | 98.6 | 11.583 |
| 120 mg/mL of anti-TSLP antibody, 20 mM of histidine buffering agent, 150 mM of arginine (26.13 g/L), 0.02% w/v of polysorbate 80, pH 6.0 | 68.29 | 63.48 | 0.44 | 98.6 | 5.092 |
| 120 mg/mL of anti-TSLP antibody, 20 mM of histidine buffering agent, 250 mM of proline (28.78 g/L), 0.02% w/v of polysorbate 80, pH 6.0 | 69.49 | 64.59 | 0.44 | 98.6 | 5.848 |

Illustratively, the histidine buffering agent was selected as the buffering agent for further screening, and the anti-TSLP antibody (hu1C5F12E9-V8 (IgG1)', having the human IgG1 heavy chain constant region set forth in SEQ ID NO: 37 and the human κ light chain constant region set forth in SEQ ID NO: 14) was displaced into the screened histidine buffering agent in Table 7 by ultrafiltration centrifugation, and after the displacement was completed, concentration was performed, and excipients were added according to the screened pharmaceutical compositions in Table 7, respectively. After mixing well, filtering and sterilizing were performed by passing through a 0.22 µm filter membrane. The appearance, visible foreign matter, biological activity, viscosity and SEC-UPLC analysis results for the different pharmaceutical compositions are shown in Table 7. The results of the stability studies for different pharmaceutical compositions at 2-8 °C are shown in Table 8.

**Table 7. Appearance, visible foreign matter, biological activity, viscosity and SEC-UPLC analysis results for the screened pharmaceutical compositions**

| Pharmaceutical composition | Appearance | Visible foreign matter | Biological activity (%) | SEC-UPLC(%) | | Viscosity (mpa·s) |
|---|---|---|---|---|---|---|
| | | | | High-mol ecular impurities | Immunoglobulin monomers | |
| 120 mg/mL of anti-TSLP antibody, 20 mM of histidine buffering agent, 200 mM of mannitol, 0.02% w/v of polysorbate 80, pH 5.5 | Colorless clear liquid | No obvious visible foreign matter | 97 | 1.22 | 96.4 | 7.060 |
| 120 mg/mL of anti-TSLP antibody, 20 mM of histidine buffering agent, 200 mM of mannitol, 0.02% w/v of polysorbate 80, pH 6.0 | Colorless clear liquid | No obvious visible foreign matter | 94 | 1.44 | 95.8 | 7.810 |
| 120 mg/mL of anti-TSLP antibody, 20 mM of histidine buffering agent, 250 mM of proline, 0.02% w/v of polysorbate 80, pH 6.0 | Colorless clear liquid | No obvious visible foreign matter | 95 | 1.23 | 96.1 | 6.652 |
| 150 mg/mL of anti-TSLP antibody, 20 mM of histidine buffering agent, 250 mM of proline, 0.02% w/v of polysorbate 80, pH 6.0 | Colorless clear liquid | No obvious visible foreign matter | 95 | 1.33 | 96.0 | 13.07 |

**Table 8. Results of stability studies for screened pharmaceutical compositions at 2-8 °C**

| Pharmaceutical composition | Test point | Appearance | Visible foreign matter | Biological activity (%) | SEC-UPLC(%) | |
|---|---|---|---|---|---|---|
| | | | | | High-mol ecular impurities | Immunoglobulin monomers |
| 120 mg/mL of anti-TSLP antibody, 20 mM of histidine buffering agent, 200 mM of mannitol, 0.02% w/v of polysorbate 80, pH 5.5 | 0 days | Colorless clear liquid | No obvious visible foreign matter | 97 | 1.22 | 96.4 |
| | Month 1 | Colorless clear liquid | No obvious visible foreign matter | 88 | 1.73 | 98.0 |
| 120 mg/mL of anti-TSLP antibody, 20 mM of histidine buffering agent, 200 mM of mannitol, 0.02% w/v of polysorbate 80, pH 6.0 | 0 days | Colorless clear liquid | No obvious visible foreign matter | 94 | 1.44 | 95.8 |
| | Month 1 | Colorless clear liquid | No obvious visible foreign matter | 101 | 1.89 | 98.0 |
| 120 mg/mL of anti-TSLP antibody, 20 mM of histidine buffering agent, 250 mM of proline, 0.02% w/v of polysorbate 80, pH 6.0 | 0 days | Colorless clear liquid | No obvious visible foreign matter | 95 | 1.23 | 96.1 |
| | Month 1 | Colorless clear liquid | No obvious visible foreign matter | 100 | 1.61 | 98.1 |
| 150 mg/mL of anti-TSLP antibody, 20 mM of histidine buffering agent, 250 mM of proline, 0.02% w/v of polysorbate 80, pH 6.0 | 0 days | Colorless clear liquid | No obvious visible foreign matter | 95 | 1.33 | 96.0 |
| | Month 1 | Colorless clear liquid | No obvious visible foreign matter | 102 | 1.84 | 98.0 |

Illustratively, the histidine buffering agent was selected as the buffering agent and proline was selected as the stabilizer for further screening, and the anti-TSLP antibody (hu1C5F12E9-V8 (IgG1)', having the human IgG1 heavy chain constant region set forth in SEQ ID NO: 37 and the human κ light chain constant region set forth in SEQ ID NO: 14) was displaced into the screened histidine buffering agent in Table 9 (acetic acid adjusted to pH 5.8) by ultrafiltration centrifugation, and after the displacement was completed, concentration was performed, and excipients were added according to the screened pharmaceutical compositions in Table 9, respectively. After mixing well, filtering and sterilizing were performed by passing through a 0.22 µm filter membrane. The viscosity, SEC-UPLC, CIEF and CE-SDS analysis results for the different pharmaceutical compositions are shown in Table 10.

**Table 9. Screened pharmaceutical compositions**

| Composition | F1 | F2 |
|---|---|---|
| Anti-TSLP antibody | 150mg/mL (150g/L) | 120mg/mL (120g/L) |
| Histidine | 20mmol/L (3.1g/L) | 20mmol/L (3.1g/L) |
| Proline | 500mmol/L (57.56g/L) | 400 mmol/L (46.05g/L) |
| Polysorbate 80 | 0.02% w/v (0.2g/L) | 0.02% w/v (0.2g/L) |
| pH | 5.8 | 5.8 |

**Table 10. Viscosity, SEC-UPLC, CIEF and CE-SDS analysis results for the screened pharmaceutical compositions**

| | | F1 | F2 |
|---|---|---|---|
| Viscosity (mpa•s) | | 13.62 | 7.51 |
| SEC-UPLC (%) | Immunoglobulin monomers | 99.0 | 99.0 |
| | High-molecular impurities | 0.5 | 0.5 |
| Capillary isoelectric focusing electrophoresis (%) | Acidic peak | 19.2 | 19.0 |
| | Main peak | 74.1 | 74.3 |
| | Basic peak | 6.7 | 6.7 |
| CE-SDS reduction electrophoresis (%) | Heavy and light chains of immunoglobulin | 98.3 | 98.1 |
| | Non-glycosylated heavy chain | 0.9 | 0.8 |
| CE-SDS non-reduction electrophoresis (%) | Immunoglobulin monomers | 96.9 | 98.2 |

Illustratively, the pharmaceutical composition F2 in Table 9 was selected for stability test at 2-8 °C, and the results of the stability studies for the pharmaceutical composition F2 at 2-8 °C are shown in Table 11.

**Table 11. Results of stability studies for the screened pharmaceutical compositions at 2-8 °C**

| | | Test time (month) | | | |
|---|---|---|---|---|---|
| | | 0 | 1 | 2 | 3 |
| Appearance | | Colorless clear liquid | Colorless clear liquid | Colorless clear liquid | Colorless clear liquid |
| Visible foreign matter | | No obvious visible foreign matter | No obvious visible foreign matter | No obvious visible foreign matter | No obvious visible foreign matter |
| SEC-UPLC (%) | Immunoglobulin monomers | 99.0 | 99.0 | 98.8 | 98.8 |
| | High-molecular impurities | 0.6 | 0.6 | 0.7 | 0.7 |
| Capillary isoelectric focusing electrophoresis (%) | Main peak | 73.9 | 72.2 | 72.1 | 71.4 |
| | Acidic peak | 19.2 | 20.2 | 20.3 | 20.4 |
| | Basic peak | 6.9 | 7.7 | 7.6 | 8.1 |
| CE-SDS reduction electrophoresis (%) | Heavy and light chains of immunoglobulin | 98.4 | 97.9 | 98.3 | 98.4 |
| | Non-glycosylated heavy chain | 0.8 | 0.8 | 0.7 | 0.7 |
| CE-SDS non-reduction | Immunoglobulin monomers | 96.6 | 96.6 | 96.9 | 96.7 |
| electrophoresis (%) | | | | | |
| Biological activity (%) | | 105 | 101 | 105 | 92 |

It should be further understood by those skilled in the art that the present disclosure can be implemented in other specific forms without departing from the spirit or key attributes thereof. In the foregoing specification, the present disclosure discloses only exemplary embodiments, and it should be understood that other variations are also encompassed within the scope of the present disclosure. Accordingly, the present disclosure is not limited to the specific embodiments described in detail herein. Rather, for the scope and content of the present disclosure, reference should be made to the appended claims.

## Claims

1. A pharmaceutical composition, comprising: (a) an anti-TSLP antibody, (b) a buffering agent, (c) a surfactant, and one or more (d) stabilizers, wherein the anti-TSLP antibody comprises a heavy chain variable region comprising a VH-CDR1 region, a VH-CDR2 region and a VH-CDR3 region, and a light chain variable region comprising a VL-CDR1 region, a VL-CDR2 region and a VL-CDR3 region, wherein the VH-CDR1 region, the VH-CDR2 region, the VH-CDR3 region, the VL-CDR1 region, the VL-CDR2 region, and the VL-CDR3 region comprise: amino acid sequences set forth in SEQ ID NOs: 1, 2, 3, 4, 5 and 6, respectively.

2. The pharmaceutical composition according to claim 1, wherein the anti-TSLP antibody comprises a heavy chain variable region and a light chain variable region, and the heavy chain variable region and the light chain variable region can comprise an amino acid sequence selected from the following amino acid sequences: (1) respectively as set forth in SEQ ID NOs: 7 and 10; (2) respectively as set forth in SEQ ID NOs: 8 and 11 (X1 = S, X2 = V); (3) respectively as set forth in SEQ ID NOs: 9 (X1 = R, X2 = V, X3 = R) and 11 (X1 = S, X2 = V); (4) respectively as set forth in SEQ ID NOs: 9 (X1 = R, X2 = V, X3 = V) and 11 (X1 = S, X2 = V); (5) respectively as set forth in SEQ ID NOs: 9 (X1 = R, X2 = A, X3 = R) and 11 (X1 = S, X2 = V); (6) respectively as set forth in SEQ ID NOs: 9 (X1 = K, X2 = A, X3 = R) and 11 (X1 = S, X2 = V); (7) respectively as set forth in SEQ ID NOs: 9 (X1 = K, X2 = A, X3 = V) and 11 (X1 = S, X2 = V); (8) respectively as set forth in SEQ ID NOs: 8 and 11 (X1 = A, X2 = I); (9) respectively as set forth in SEQ ID NOs: 9 (X1 = R, X2 = V, X3 = R) and 11 (X1 = A, X2 = I); (10) respectively as set forth in SEQ ID NOs: 9 (X1 = R, X2 = V, X3 = V) and 11 (X1 = A, X2 = I); (11) respectively as set forth in SEQ ID NOs: 9 (X1 = R, X2 = A, X3 = R) and 11 (X1 = A, X2 = I); (12) respectively as set forth in SEQ ID NOs: 9 (X1 = K, X2 = A, X3 = R) and 11 (X1 = A, X2 = I); (13) respectively as set forth in SEQ ID NOs: 9 (X1 = K, X2 = A, X3 = V) and 11 (X1 = A, X2 = I); (14) respectively as set forth in SEQ ID NOs: 8 and 11 (X1 = S, X2 = I); (15) respectively as set forth in SEQ ID NOs: 9 (X1 = R, X2 = V, X3 = R) and 11 (X1 = S, X2 = I); (16) respectively as set forth in SEQ ID NOs: 9 (X1 = R, X2 = V, X3 = V) and 11 (X1 = S, X2 = I); (17) respectively as set forth in SEQ ID NOs: 9 (X1 = R, X2 = A, X3 = R) and 11 (X1 = S, X2 = I); (18) respectively as set forth in SEQ ID NOs: 9 (X1 = K, X2 = A, X3 = R) and 11 (X1 = S, X2 = I); or (19) respectively as set forth in SEQ ID NOs: 9 (X1 = K, X2 = A, X3 = V) and 11 (X1 = S, X2 = I).

3. The pharmaceutical composition according to any one of claims 1-2, wherein the anti-TSLP antibody has a concentration of 30 mg/mL to 300 mg/mL, preferably 50 mg/mL to 250 mg/mL, preferably 70 mg/mL to 200 mg/mL, more preferably 90 mg/mL to 150 mg/mL, most preferably 120 mg/mL to 150 mg/mL.

4. The pharmaceutical composition according to any one of claims 1-3, wherein the buffering agent comprises a phosphate buffering agent, a histidine buffering agent, an acetate buffering agent, or a citrate buffering agent.

5. The pharmaceutical composition according to claim 4, wherein the phosphate buffering agent is a sodium phosphate buffering agent, the acetate buffering agent is a sodium acetate buffering agent, and the citrate buffering agent is a sodium citrate buffering agent.

6. The pharmaceutical composition according to any one of claims 1-5, wherein the buffering agent has a concentration of 1 mM to 100 mM, preferably 2 mM to 80 mM, more preferably 5 mM to 60 mM, most preferably 10 mM to 40 mM.

7. The pharmaceutical composition according to any one of claims 1-6, wherein the surfactant comprises polysorbate 80 or polysorbate 20.

8. The pharmaceutical composition according to any one of claims 1-7, wherein the surfactant has a concentration of 0.001% (w/v) to 0.1% (w/v), preferably 0.004% (w/v) to 0.08% (w/v), more preferably 0.006% (w/v) to 0.06% (w/v), most preferably 0.008% (w/v) to 0.04% (w/v).

9. The pharmaceutical composition according to any one of claims 1-8, wherein the stabilizer comprises trehalose, mannitol, sucrose, arginine or a pharmaceutically acceptable salt thereof, glycine, or proline.

10. The pharmaceutical composition according to any one of claims 1-9, wherein the stabilizer has a concentration of 100 mM to 1000 mM, preferably 120 mM to 800 mM, more preferably 150 mM to 700 mM, most preferably 150 mM to 600 mM.

11. The pharmaceutical composition according to any one of claims 1-10, wherein the pharmaceutical composition has a pH of 5 to 7, preferably 5.5 to 7, more preferably 5.5 to 6.5.

12. A lyophilized formulation comprising an anti-TSLP antibody, the lyophilized formulation being obtained by lyophilizing the pharmaceutical composition according to any one of claims 1-11.

13. A lyophilized formulation comprising an anti-TSLP antibody, the lyophilized formulation being capable of forming the pharmaceutical composition according to any one of claims 1-11 upon reconstitution.

14. An article of manufacture, comprising a container comprising the pharmaceutical composition according to any one of claims 1-11, or the lyophilized formulation according to any one of claims 12-13.

15. A method for treating and preventing TSLP-related diseases, comprising administering to a patient in need thereof a therapeutically effective amount of the pharmaceutical composition according to any one of claims 1-11 or the lyophilized formulation according to any one of claims 12-13 or the article of manufacture according to claim 14.
